# EUROPÄISCHE PATENTSCHRIFT

(11) **EP 3 433 360 B1**
(45) Veröffentlichungstag und Bekanntmachung des Hinweises auf die Patenterteilung: **16.09.2020**
(21) Anmeldenummer: 17709422.4
(22) Anmeldetag: 07.03.2017
(51) Int. Cl.: C12N 9/54, C11D 3/386

(54) **PROTEASEN MIT VERBESSERTE ENZYMSTABILITÄT IN WASCHMITTEL**
PROTEASES WITH IMPROVED ENZYME STABILITY IN DETERGENTS
PROTÉASES PRÉSENTANT UNE MEILLEURE STABILITÉ ENZYMATIQUE DANS LES DÉTERGENTS

(30) Priorität: 23.03.2016 DE 102016204815
(43) Veröffentlichungstag der Anmeldung: 30.01.2019
(73) Patentinhaber: Henkel AG & Co. KGaA, 40589 Düsseldorf (DE)
(72) Erfinder: MUSSMANN, Nina, 47877 Willich (DE); O'CONNELL, Timothy, 86899 Landsberg am Lech (DE); HERBST, Daniela, 40211 Düsseldorf (DE); PRÜSER, Inken, 40215 Düsseldorf (DE); DEGERING, Christian, 40699 Erkrath (DE); GRIEMERT, Sabine, 40789 Monheim am Rhein (DE); EGGERT, Thorsten, 45529 Hattingen (DE); LEGGEWIE, Christian, 40789 Monheim am Rhein (DE)
(86) Internationale Anmeldenummer: PCT/EP2017/055277
(87) Internationale Veröffentlichungsnummer: WO 2017/162429

(56) Entgegenhaltungen:
- WO-A1-95/30010
- WO-A1-2010/060822
- WO-A1-2011/014278
- WO-A1-2011/036263
- WO-A1-2011/036264
- WO-A1-2011/072117
- WO-A1-2014/207228
- WO-A1-2015/089441
- WO-A2-96/28557
- WO-A2-96/28566
- DE-A1-102006 022 224
- US-A- 5 741 664
- US-A1- 2003 191 038
- PHILIP N. BRYAN: "Protein engineering of subtilisin", BIOCHIMICA ET BIOPHYSICA ACTA. PROTEIN STRUCTURE AND MOLECULAR ENZYMOLOGY, Bd. 1543, Nr. 2, 1. Dezember 2000 (2000-12-01), Seiten 203-222, XP055251064, AMSTERDAM; NL ISSN: 0167-4838, DOI: 10.1016/S0167-4838(00)00235-1
- DATABASE UniProt [Online] 14. Oktober 2015 (2015-10-14), "SubName: Full=Serine neutral keratinase preproprotein {ECO:0000313|EMBL:AKN20219.1};", XP002769925, gefunden im EBI accession no. UNIPROT:A0A0H3YE38 Database accession no. A0A0H3YE38
- DATABASE UniProt [Online] 29. Mai 2013 (2013-05-29), "SubName: Full=Protease {ECO:0000313|EMBL:BAN09121.1};", XP002769926, gefunden im EBI accession no. UNIPROT:M5B284 Database accession no. M5B284
- DATABASE UniProt [Online] 4. April 2006 (2006-04-04), "SubName: Full=Subtilisin-like serine proteinase {ECO:0000313|EMBL:AAX14553.1};", XP002769868, gefunden im EBI accession no. UNIPROT:Q29ZA8 Database accession no. Q29ZA8
- DATABASE UniProt [Online] 21. Juli 1986 (1986-07-21), "RecName: Full=Subtilisin Carlsberg; EC=3.4.21.62; Flags: Precursor;", XP002769870, gefunden im EBI accession no. UNIPROT:P00780 Database accession no. P00780
- DATABASE UniProt [Online] 23. September 2008 (2008-09-23), "SubName: Full=Subtilisin Carlsberg {ECO:0000313|EMBL:EDW22774.1}; EC=3.4.21.62 {ECO:0000313|EMBL:EDW22774.1};", XP002769869, gefunden im EBI accession no. UNIPROT:B4AFK7 Database accession no. B4AFK7

## Beschreibung

Die Erfindung liegt auf dem Gebiet der Enzymtechnologie. Die Erfindung betrifft Proteasen aus *Bacillus pumilus* deren Aminosäuresequenz, insbesondere im Hinblick auf den Einsatz in Wasch- und Reinigungsmitteln, verändert wurde, um ihnen eine bessere Lagerstabilität zu verleihen, und die für sie kodierende Nukleinsäuren sowie deren Herstellung. Die Erfindung betrifft ferner die Verwendungen dieser Proteasen und Verfahren, in denen sie eingesetzt werden, sowie die Proteasen enthaltende Mittel, insbesondere Wasch- und Reinigungsmittel.

Proteasen gehören zu den technisch bedeutendsten Enzymen überhaupt. Für Wasch- und Reinigungsmittel sind sie die am längsten etablierten und in praktisch allen modernen, leistungsfähigen Wasch- und Reinigungsmitteln enthaltenen Enzyme. Sie bewirken den Abbau proteinhaltiger Anschmutzungen auf dem Reinigungsgut. Hierunter sind wiederum Proteasen vom Subtilisin-Typ (Subtilasen, Subtilopeptidasen, EC 3.4.21.62) besonders wichtig, welche aufgrund der katalytisch wirksamen Aminosäuren Serin-Proteasen sind. Sie wirken als unspezifische Endopeptidasen und hydrolysieren beliebige Säureamidbindungen, die im Inneren von Peptiden oder Proteinen liegen. Ihr pH-Optimum liegt meist im deutlich alkalischen Bereich. Einen Überblick über diese Familie bietet beispielsweise der Artikel "Subtilases: Subtilisin-like Proteases" von R. Siezen, Seite 75-95 in "Subtilisin enzymes", herausgegeben von R. Bott und C. Betzel, New York, 1996. Subtilasen werden natürlicherweise von Mikroorganismen gebildet. Hierunter sind insbesondere die von *Bacillus-*Spezies gebildeten und sezernierten Subtilisine als bedeutendste Gruppe innerhalb der Subtilasen zu erwähnen.

Beispiele für die in Wasch- und Reinigungsmitteln bevorzugt eingesetzten Proteasen vom Subtilisin-Typ sind die Subtilisine BPN' und Carlsberg, die Protease PB92, die Subtilisine 147 und 309, die Protease aus *Bacillus lentus,* insbesondere aus *Bacillus lentus* DSM 5483, Subtilisin DY und die den Subtilasen, nicht mehr jedoch den Subtilisinen im engeren Sinne zuzuordnenden Enzyme Thermitase, Proteinase K und die Proteasen TW3 und TW7, sowie Varianten der genannten Proteasen, die eine gegenüber der Ausgangsprotease veränderte Aminosäuresequenz aufweisen. Proteasen werden durch aus dem Stand der Technik bekannte Verfahren gezielt oder zufallsbasiert verändert und so beispielsweise für den Einsatz in Wasch- und Reinigungsmitteln optimiert. Dazu gehören Punktmutagenese, Deletions- oder Insertionsmutagenese oder Fusion mit anderen

### Beschreibung (Reinschrift)

Proteinen oder Proteinteilen. So sind für die meisten aus dem Stand der Technik bekannten Proteasen entsprechend optimierte Varianten bekannt. Aus z.B. WO9530010, US5741664A, WO2011014278, WO2011036263, WO2011036264, WO2014207228, WO2011072177, US2003191038A1 sind eine Reihe von BPN' und Savinase Varianten bekannt. Weitere Subtilisine sind aus z.B. WO9628566, WO2015089441 und WO9628557 bekannt. WO2010060822 und DE102006022224 offenbaren eine alkalische Protease aus Bacillus pumilus.
In der europäischen Patentanmeldung EP 2 016 175 A1 ist beispielsweise eine für Wasch- und Reinigungsmittel vorgesehene Protease aus *Bacillus pumilus* offenbart. Generell sind nur ausgewählte Proteasen für den Einsatz in flüssigen Tensid-haltigen Zubereitungen überhaupt geeignet. Viele Proteasen zeigen in derartigen Zubereitungen keine ausreichende katalytische Leistung oder Stabilität. Insbesondere bei der Verwendung in Waschmitteln, die normalerweise in einer Menge vom Verbraucher bezogen werden, so dass bis zum endgültigen Verbrauch des Waschmittels mehrere Wochen oder Monate vergehen können (d.h. die Waschmittel müssen vom Verbraucher über Wochen oder Monate gelagert werden) zeigen viele Proteasen eine Instabilität, die wiederum zu einer unzureichenden katalytischen Aktivität während des Waschvorgangs führt. In Phosphonat-haltigen flüssigen Tensidzubereitungen ist diese Problematik noch gravierender, beispielsweise auf Grund der komplexbildenden Eigenschaften der Phosphonate oder auf Grund von unvorteilhaften Wechselwirkungen zwischen dem Phosphonat und der Protease.

Folglich haben Protease- und Tensid-haltige flüssige Formulierungen aus dem Stand der Technik den Nachteil, dass sie oftmals nach Lagerung der Formulierung keine zufriedenstellende proteolytische Aktivität aufweisen und daher keine optimale Reinigungsleistung an Proteasesensitiven Anschmutzungen zeigen.

Überraschenderweise wurde jetzt festgestellt, dass eine Protease aus *Bacillus pumilus* oder eine hierzu hinreichend ähnliche Protease (bezogen auf die Sequenzidentität), die eine Aminosäuresubstitution an mindestens einer der Positionen P9, Q62, D101, N130, G166, N187, S216, N238 oder Q271, jeweils bezogen auf die Nummerierung gemäß SEQ ID NO:2, aufweist, hinsichtlich der (Lager-)Stabilität gegenüber der Wildtypform verbessert ist und daher besonders für den Einsatz in Wasch- oder Reinigungsmitteln geeignet ist.

Gegenstand der Erfindung ist daher in einem ersten Aspekt eine Protease umfassend eine Aminosäuresequenz, die mindestens 90% Sequenzidentität mit der in SEQ ID NO:2 angegebenen Aminosäuresequenz über deren Gesamtlänge aufweist und eine Aminosäuresubstitution an mindestens einer der Positionen P9, Q62, D101, N130, G166, N187, S216, N238 oder Q271, jeweils bezogen auf die Nummerierung gemäß SEQ ID NO:2, aufweist, wobei die mindestens eine Aminosäuresubstitution aus der Gruppe bestehend aus P9T/S/H, Q62E, D101E, N130D, G166S, N187H, S216C, N238K oder Q271E, jeweils bezogen auf die Nummerierung gemäß SEQ ID NO:2, ausgewählt ist.

Ein weiterer Gegenstand der Erfindung ist ein Verfahren zur Herstellung einer Protease umfassend das Substituieren einer Aminosäure an mindestens einer Position, die der Position 9, 62, 101, 130, 166, 187, 216, 238 oder 271 in SEQ ID NO:2 entspricht, in einer Ausgangsprotease, die mindestens 90% Sequenzidentität zu der in SEQ ID NO:2 angegebenen Aminosäuresequenz über deren Gesamtlänge aufweist, derart, dass die Protease mindestens eine der Aminosäuresubstitutionen P9T/S/H, Q62E, D101E, N130D, G166S, N187H, S216C, N238K oder Q271E aufweist.

Eine Protease im Sinne der vorliegenden Patentanmeldung umfasst daher sowohl die Protease als solche als auch eine mit einem erfindungsgemäßen Verfahren hergestellte Protease. Alle Ausführungen zur Protease beziehen sich daher sowohl auf die Protease als solche wie auch auf die mittels entsprechender Verfahren hergestellten Proteasen.

Weitere Aspekte der Erfindung betreffen die für diese Proteasen kodierenden Nukleinsäuren, erfindungsgemäße Proteasen oder Nukleinsäuren enthaltende nicht menschliche Wirtszellen sowie erfindungsgemäße Proteasen umfassende Mittel, insbesondere Wasch- und Reinigungsmittel, Wasch- und Reinigungsverfahren, und Verwendungen der erfindungsgemäßen Proteasen in Wasch- oder Reinigungsmitteln zur Entfernung von fetthaltigen Anschmutzungen.

"Mindestens eine", wie hierin verwendet, bedeutete eine oder mehrere, d.h. 1, 2, 3, 4, 5, 6, 7, 8, 9, 10, 11, 12, 13, 14 oder mehr.

Die vorliegende Erfindung basiert auf der überraschenden Erkenntnis der Erfinder, dass eine Aminosäuresubstitution an mindestens einer der Positionen 9, 62, 101, 130, 166, 187, 216, 238 oder 271 der Protease aus *Bacillus pumilus* gemäß SEQ ID NO:2, in einer Protease, die eine zu der in SEQ ID NO:2 angegebenen Aminosäuresequenz zu mindestens 90% identische Aminosäuresequenz umfasst, derart, dass an mindestens einer der entsprechenden Positionen die Aminosäuren 9T/S/H, 62E, 101E, 130D, 166S, 187H, 216C, 238K oder 271E vorhanden sind, eine verbesserte (Lager-)Stabilität dieser veränderten Protease in Wasch- und Reinigungsmitteln bewirkt. Das ist insbesondere insoweit überraschend, als dass keine der oben genannten Aminosäuresubstitutionen zuvor mit einer erhöhten Stabilität der Protease in Verbindung gebracht wurde.

Die erfindungsgemäßen Proteasen verfügen über eine erhöhte Stabilität in Wasch- oder Reinigungsmitteln, insbesondere bei einer Lagerung von 3 oder mehr Tagen, 4 oder mehr Tagen, 7 oder mehr Tagen, 10 oder mehr Tagen, 12 oder mehr Tagen oder 14 oder mehr Tagen. Solche leistungsverbesserten Proteasen ermöglichen verbesserte Waschergebnisse an proteolytischsensitiven Anschmutzungen in einem weiten Temperaturbereich.

Die erfindungsgemäßen Proteasen weisen enzymatische Aktivität auf, das heißt, sie sind zur Hydrolyse von Peptiden und Proteinen befähigt, insbesondere in einem Wasch- oder Reinigungsmittel. Eine erfindungsgemäße Protease ist daher ein Enzym, welches die Hydrolyse von Amid/Peptidbindungen in Protein/Peptid-Substraten katalysiert und dadurch in der Lage ist, Proteine oder Peptide zu spalten. Ferner handelt es sich bei einer erfindungsgemäßen Protease vorzugsweise um eine reife (mature) Protease, d.h. um das katalytisch aktive Molekül ohne Signal- und/oder Propeptid(e). Soweit nicht anders angegeben beziehen sich auch die angegebenen Sequenzen auf jeweils reife (prozessierte) Enzyme.

In verschiedenen Ausführungsformen enthält die erfindungsgemäße Protease mindestens eine Aminosäuresubstitution, die aus der Gruppe bestehend aus P9T/S/H, Q62E, D101E, N130D, G166S, N187H, S216C, N238K und Q271E, jeweils bezogen auf die Nummerierung gemäß SEQ ID NO:2, ausgewählt ist. In weiter bevorzugten Ausführungsformen enthält die erfindungsgemäße Protease eine der folgenden Aminosäuresubstitutionsvarianten: (i) P9T; (ii) Q271E; (iii) P9S und N238K; (iv) P9H; (v) N187H; (vi) S216C; (vii) Q62E und N130D; (viii) Q62E, D101E, N130D, G166S und Q271E; oder (ix) P9T, N187H, S216C und Q271E, wobei die Nummerierung jeweils bezogen ist auf die Nummerierung gemäß SEQ ID NO:2.

In einer weiteren Ausführungsform der Erfindung umfasst die Protease eine Aminosäuresequenz, die zu der in SEQ ID NO:2 angegebenen Aminosäuresequenz über deren Gesamtlänge zu mindestens 90%, 90,5%, 91%, 91,5%, 92%, 92,5%, 93%, 93,5%, 94%, 94,5%, 95%, 95,5%, 96%, 96,5%, 97%, 97,5%, 98%, 98,5% und 98,8% identisch ist, und die an mindestens einer der Positionen 9, 62, 101, 130, 166, 187, 216, 238 oder 271 in der Zählung gemäß SEQ ID NO:2, eine oder mehrere der Aminosäuresubstitutionen 9T/S/H, 62E, 101E, 130D, 166S, 187H, 216C, 238K oder 271E aufweist. Im Zusammenhang mit der vorliegenden Erfindung bedeutet das Merkmal, dass eine Protease die angegebenen Substitutionen aufweist, dass sie mindestens eine der entsprechenden Aminosäuren an den entsprechenden Positionen enthält, d.h. nicht alle der 9 Positionen anderweitig mutiert oder, beispielsweise durch Fragmentierung der Protease, deletiert sind. Die Aminosäuresequenzen derartiger Proteasen, die erfindungsgemäß bevorzugt sind, sind in SEQ ID Nos: 3-11 angegeben.

Die Bestimmung der Identität von Nukleinsäure- oder Aminosäuresequenzen erfolgt durch einen Sequenzvergleich. Dieser Sequenzvergleich basiert auf dem im Stand der Technik etablierten und üblicherweise genutzten BLAST-Algorithmus (vgl. beispielsweise Altschul, S.F., Gish, W., Miller, W., Myers, E.W. & Lipman, D.J. (1990) "Basic local alignment search tool." J. Mol. Biol. 215:403-410, und Altschul, Stephan F., Thomas L. Madden, Alejandro A. Schaffer, Jinghui Zhang, Hheng Zhang, Webb Miller, and David J. Lipman (1997): "Gapped BLAST and PSI-BLAST: a new generation of protein database search programs"; Nucleic Acids Res., 25, S.3389-3402) und geschieht prinzipiell dadurch, dass ähnliche Abfolgen von Nukleotiden oder Aminosäuren in den Nukleinsäure- oder Aminosäuresequenzen einander zugeordnet werden. Eine tabellarische Zuordnung der betreffenden Positionen wird als Alignment bezeichnet. Ein weiterer im Stand der Technik verfügbarer Algorithmus ist der FASTA-Algorithmus. Sequenzvergleiche (Alignments), insbesondere multiple Sequenzvergleiche, werden mit Computerprogrammen erstellt. Häufig genutzt werden beispielsweise die Clustal-Serie (vgl. beispielsweise Chenna et al. (2003): Multiple sequence alignment with the Clustal series of programs. Nucleic Acid Research 31, 3497-3500), T-Coffee (vgl. beispielsweise Notredame et al. (2000): T-Coffee: A novel method for multiple sequence alignments. J. Mol. Biol. 302, 205-217) oder Programme, die auf diesen Programmen beziehungsweise Algorithmen basieren. Ferner möglich sind Sequenzvergleiche (Alignments) mit dem Computer-Programm Vector NTI® Suite 10.3 (Invitrogen Corporation, 1600 Faraday Avenue, Carlsbad, Kalifornien, USA) mit den vorgegebenen Standardparametern, dessen AlignX-Modul für die Sequenzvergleiche auf ClustalW basiert. Soweit nicht anders angegeben, wird die hierin angegebene Sequenzidentität mit dem BLAST-Algorithmus bestimmt.

Solch ein Vergleich erlaubt auch eine Aussage über die Ähnlichkeit der verglichenen Sequenzen zueinander. Sie wird üblicherweise in Prozent Identität, das heißt dem Anteil der identischen Nukleotide oder Aminosäurereste an denselben oder in einem Alignment einander entsprechenden Positionen angegeben. Der weiter gefasste Begriff der Homologie bezieht bei Aminosäuresequenzen konservierte Aminosäure-Austausche in die Betrachtung mit ein, also Aminosäuren mit ähnlicher chemischer Aktivität, da diese innerhalb des Proteins meist ähnliche chemische Aktivitäten ausüben. Daher kann die Ähnlichkeit der verglichenen Sequenzen auch Prozent Homologie oder Prozent Ähnlichkeit angegeben sein. Identitäts- und/oder Homologieangaben können über ganze Polypeptide oder Gene oder nur über einzelne Bereiche getroffen werden. Homologe oder identische Bereiche von verschiedenen Nukleinsäure- oder Aminosäuresequenzen sind daher durch Übereinstimmungen in den Sequenzen definiert. Solche Bereiche weisen oftmals identische Funktionen auf. Sie können klein sein und nur wenige Nukleotide oder Aminosäuren umfassen. Oftmals üben solche kleinen Bereiche für die Gesamtaktivität des Proteins essentielle Funktionen aus. Es kann daher sinnvoll sein, Sequenzübereinstimmungen nur auf einzelne, gegebenenfalls kleine Bereiche zu beziehen. Soweit nicht anders angegeben beziehen sich Identitäts- oder Homologieangaben in der vorliegenden Anmeldung aber auf die Gesamtlänge der jeweils angegebenen Nukleinsäure- oder Aminosäuresäuresequenz.

Im Zusammenhang mit der vorliegenden Erfindung bedeutet die Angabe, dass eine Aminosäurepositon einer numerisch bezeichneten Position in SEQ ID NO:2 entspricht daher, dass die entsprechende Position der numerisch bezeichneten Position in SEQ ID NO:2 in einem wie oben definierten Alignment zugeordnet ist.

In einer weiteren Ausführungsform der Erfindung ist die Protease dadurch gekennzeichnet, dass ihre Reinigungsleistung gegenüber derjenigen einer Protease, die eine Aminosäuresequenz umfasst, die der in SEQ ID NO:2 angegebenen Aminosäuresequenz entspricht, nicht signifikant verringert ist, d.h. mindestens 80 % der Referenzwaschleistung besitzt, vorzugsweise mindestens 100 %, noch bevorzugter mindestens 110%. Die Reinigungsleistung kann in einem Waschsystem bestimmt werden, das ein Waschmittel in einer Dosierung zwischen 4,5 und 7,0 Gramm pro Liter Waschflotte sowie die Protease enthält, wobei die zu vergleichenden Proteasen konzentrationsgleich (bezogen auf aktives Protein) eingesetzt sind und die Reinigungsleistung gegenüber einer Anschmutzung auf Baumwolle bestimmt wird durch Messung des Reinigungsgrades der gewaschenen Textilien. Beispielsweise kann der Waschvorgang für 70 Minuten bei einer Temperatur von 40°C erfolgen und das Wasser eine Wasserhärte zwischen 15,5 und 16,5° (deutsche Härte) aufweisen. Die Konzentration der Protease in dem für dieses Waschsystem bestimmten Waschmittel beträgt 0,001-0,1 Gew.-%, vorzugsweise 0,01 bis 0,06 Gew.-%, bezogen auf aktives, gereinigtes Protein.

Ein flüssiges Referenzwaschmittel für ein solches Waschsystem kann wie folgt zusammengesetzt sein (alle Angaben in Gewichts-Prozent): 4,4% Alkylbenzolsulfonsäure, 5,6% weitere anionische Tenside, 2,4% C12-C18 Na-Salze von Fettsäuren (Seifen), 4,4% nicht-ionische Tenside, 0,2% Phosphonate, 1,4% Zitronensäure, 0,95% NaOH, 0,01% Entschäumer, 2% Glycerin, 0,08% Konservierungsstoffe, 1% Ethanol, Rest demineralisiertes Wasser. Bevorzugt beträgt die Dosierung des flüssigen Waschmittels zwischen 4,5 und 6,0 Gramm pro Liter Waschflotte, beispielsweise 4,7, 4,9 oder 5,9 Gramm pro Liter Waschflotte. Bevorzugt wird gewaschen in einem pH-Wertebereich zwischen pH 8 und pH 10,5, bevorzugt zwischen pH 8 und pH 9.

Im Rahmen der Erfindung erfolgt die Bestimmung der Reinigungsleistung beispielsweise bei 40°C unter Verwendung eines flüssigen Waschmittels wie vorstehend angegeben, wobei der Waschvorgang vorzugsweise für 60 Minuten bei 600 rpm erfolgt.

Der Weißegrad, d.h. die Aufhellung der Anschmutzungen, als Maß für die Reinigungsleistung wird mit optischen Messverfahren bestimmt, bevorzugt photometrisch. Ein hierfür geeignetes Gerät ist beispielsweise das Spektrometer Minolta CM508d. Üblicherweise werden die für die Messung eingesetzten Geräte zuvor mit einem Weißstandard, bevorzugt einem mitgelieferten Weißstandard, kalibriert.

Durch den aktivitätsgleichen Einsatz der jeweiligen Protease wird sichergestellt, dass auch bei einem etwaigen Auseinanderklaffen des Verhältnisses von Aktivsubstanz zu Gesamtprotein (die Werte der spezifischen Aktivität) die jeweiligen enzymatischen Eigenschaften, also beispielsweise die Reinigungsleistung an bestimmten Anschmutzungen, verglichen werden. Generell gilt, dass eine niedrige spezifische Aktivität durch Zugabe einer größeren Proteinmenge ausgeglichen werden kann.

Ansonsten sind Verfahren zur Bestimmung der Proteaseaktivität dem Fachmann auf dem Gebiet der Enzymtechnologie geläufig und werden von ihm routinemäßig angewendet. Beispielsweise sind solche Verfahren offenbart in Tenside, Band 7 (1970), S. 125-132. Alternativ kann die Protease-Aktivität über die Freisetzung des Chromophors para-Nitroanilin (pNA) aus dem Substrat suc-L-Ala-L-Ala-L-Pro-L-Phe-p-Nitroanilid (AAPF) bestimmt werden. Die Protease spaltet das Substrat und setzt pNA frei. Die Freisetzung des pNA verursacht eine Zunahme der Extinktion bei 410 nm, deren zeitlicher Verlauf ein Maß für die enzymatische Aktivität ist (vgl. Del Mar et al., 1979). Die Messung erfolgt bei einer Temperatur von 25°C, bei pH 8,6, und einer Wellenlänge von 410 nm. Die Messzeit beträgt 5 min und das Messintervall 20s bis 60s. Die Proteaseaktivität wird üblicherweise in Protease-Einheiten (PE) angegeben. Geeignete Proteaseaktivitäten betragen beispielsweise 2,25, 5 oder 10 PE pro ml Waschflotte. Die Proteaseaktivität ist jedoch nicht gleich Null.

Ein alternativer Test zur Feststellung der proteolytischen Aktivität der erfindungsgemäßen Proteasen ist ein optisches Messverfahren, bevorzugt ein photometrisches Verfahren. Der hierfür geeignete Test umfasst die Protease-abhängige Spaltung des Substratproteins Casein. Dieses wird durch die Protease in eine Vielzahl kleinerer Teilprodukte gespalten. Die Gesamtheit dieser Teilprodukte weist eine erhöhte Absorption bei 290 nm gegenüber nicht gespaltenem Casein auf, wobei diese erhöhte Absorption unter Verwendung eines Photometers ermittelt werden und somit ein Rückschluss auf die enzymatische Aktivität der Protease gezogen werden kann.

Die Proteinkonzentration kann mit Hilfe bekannter Methoden, zum Beispiel dem BCA-Verfahren (Bicinchoninsäure; 2,2'-Bichinolyl-4,4'-dicarbonsäure) oder dem Biuret-Verfahren (A. G. Gornall, C. S. Bardawill und M.M. David, J. Biol. Chem., 177 (1948), S. 751-766) bestimmt werden. Die Bestimmung der Aktivproteinkonzentration kann diesbezüglich über eine Titration der aktiven Zentren unter Verwendung eines geeigneten irreversiblen Inhibitors und Bestimmung der Restaktivität (vgl. M. Bender et al., J. Am. Chem. Soc. 88, 24 (1966), S. 5890-5913) erfolgen.

Zusätzlich zu den vorstehend erläuterten Aminosäureveränderungen können erfindungsgemäße Proteasen weitere Aminosäureveränderungen, insbesondere Aminosäure-Substitutionen, -Insertionen oder -Deletionen, aufweisen. Solche Proteasen sind beispielsweise durch gezielte genetische Veränderung, d.h. durch Mutageneseverfahren, weiterentwickelt und für bestimmte Einsatzzwecke oder hinsichtlich spezieller Eigenschaften (beispielsweise hinsichtlich ihrer katalytischen Aktivität, Stabilität, usw.) optimiert. Ferner können erfindungsgemäße Nukleinsäuren in Rekombinationsansätze eingebracht und damit zur Erzeugung völlig neuartiger Proteasen oder anderer Polypeptide genutzt werden.

Das Ziel ist es, in die bekannten Moleküle gezielte Mutationen wie Substitutionen, Insertionen oder Deletionen einzuführen, um beispielsweise die Reinigungsleistung von erfindungsgemäßen Enzymen zu verbessern. Hierzu können insbesondere die Oberflächenladungen und/oder der isoelektrische Punkt der Moleküle und dadurch ihre Wechselwirkungen mit dem Substrat verändert werden. So kann beispielsweise die Nettoladung der Enzyme verändert werden, um darüber die Substratbindung insbesondere für den Einsatz in Wasch- und Reinigungsmitteln zu beeinflussen. Alternativ oder ergänzend kann durch eine oder mehrere entsprechende Mutationen die Stabilität der Protease noch weiter erhöht und dadurch ihre Reinigungsleistung verbessert werden. Vorteilhafte Eigenschaften einzelner Mutationen, z.B. einzelner Substitutionen, können sich ergänzen. Eine hinsichtlich bestimmter Eigenschaften bereits optimierter Proteasen, zum Beispiel hinsichtlich ihrer Stabilität bei Lagerung, kann daher im Rahmen der Erfindung zusätzlich weiterentwickelt sein.

Für die Beschreibung von Substitutionen, die genau eine Aminosäureposition betreffen (Aminosäureaustausche), wird hierin folgende Konvention angewendet: zunächst wird die natürlicherweise vorhandene Aminosäure in Form des international gebräuchlichen Einbuchstaben-Codes bezeichnet, dann folgt die zugehörige Sequenzposition und schließlich die eingefügte Aminosäure. Wenn eine Position durch mehrere alternative Aminosäuren ersetzt werden kann, werden die Alternativen durch Schrägstriche voneinander getrennt. So bedeutet beispielsweise P9T/S/H, dass Prolin an Position 9 durch Threonin, Serin oder Histidin ersetzt sein kann. Bei Insertionen sind nach der Sequenzposition zusätzliche Aminosäuren benannt. Bei Deletionen ist die fehlende Aminosäure durch ein Symbol, beispielsweise einen Stern oder einen Strich, ersetzt oder vor der entsprechenden Position ein Δ angegeben. Beispielsweise beschreibt P9T die Substitution von Prolin an Position 9 durch Threonin, P9KT die Insertion von Threonin nach der Aminosäure Lysin an Position 9 und P9* oder ΔP9 die Deletion von Prolin an Position 9. Diese Nomenklatur ist dem Fachmann auf dem Gebiet der Enzymtechnologie bekannt.

Ein weiterer Gegenstand der Erfindung ist daher eine Protease, die dadurch gekennzeichnet ist, dass sie aus einer Protease wie vorstehend beschrieben als Ausgangsmolekül erhältlich ist durch ein- oder mehrfache konservative Aminosäuresubstitution, wobei die Protease in der Zählung gemäß SEQ ID NO:2 noch mindestens eine der erfindungsgemäßen Aminosäuresubstitutionen an den Positionen, die Positionen 9, 62, 101, 130, 166, 187, 216, 238 und 271 in SEQ ID NO:2 entsprechen, aufweist, wie vorstehend beschrieben. Der Begriff "konservative Aminosäuresubstitution" bedeutet den Austausch (Substitution) eines Aminosäurerestes gegen einen anderen Aminosäurerest, wobei dieser Austausch nicht zu einer Änderung der Polarität oder Ladung an der Position der ausgetauschten Aminosäure führt, z. B. der Austausch eines unpolaren Aminosäurerestes gegen einen anderen unpolaren Aminosäurerest. Konservative Aminosäuresubstitutionen im Rahmen der Erfindung umfassen beispielsweise: G=A=S, I=V=L=M, D=E, N=Q, K=R, Y=F, S=T, G=A=I=V=L=M=Y=F=W=P=S=T.

Alternativ oder ergänzend ist die Protease dadurch gekennzeichnet, dass sie aus einer erfindungsgemäßen Protease als Ausgangsmolekül erhältlich ist durch Fragmentierung, Deletions-, Insertions- oder Substitutionsmutagenese und eine Aminosäuresequenz umfasst, die über eine Länge von mindestens 50, 60, 70, 80, 90, 100, 110, 120, 130, 140, 150, 160, 170, 180, 190, 200, 210, 220, 230, 240, 250, 260, 265, 270, 271, 272, 273 oder 274 zusammenhängenden Aminosäuren mit dem Ausgangsmolekül übereinstimmt, wobei die im Ausgangsmolekül enthaltene(n) Aminosäuresubstitution(en) an einer oder mehreren der Positionen, die Positionen 9, 62, 101, 130, 166, 187, 216, 238 und 271 in SEQ ID NO:2 entsprechen, noch vorhanden ist/sind.

So ist es beispielsweise möglich, an den Termini oder in den Loops des Enzyms einzelne Aminosäuren zu deletieren, ohne dass dadurch die proteolytische Aktivität verloren oder vermindert wird. Ferner kann durch derartige Fragmentierung, Deletions-, Insertions- oder Substitutionsmutagenese beispielsweise auch die Allergenizität betreffender Enzyme gesenkt und somit insgesamt ihre Einsetzbarkeit verbessert werden. Vorteilhafterweise behalten die Enzyme auch nach der Mutagenese ihre proteolytische Aktivität, d.h. ihre proteolytische Aktivität entspricht mindestens derjenigen des Ausgangsenzyms, d.h. in einer bevorzugten Ausführungsform beträgt die proteolytische Aktivität mindestens 80, vorzugsweise mindestens 90 % der Aktivität des Ausgangsenzyms. Auch weitere Substitutionen können vorteilhafte Wirkungen zeigen. Sowohl einzelne wie auch mehrere zusammenhängende Aminosäuren können gegen andere Aminosäuren ausgetauscht werden.

Alternativ oder ergänzend ist die Protease dadurch gekennzeichnet, dass sie aus einer erfindungsgemäßen Protease als Ausgangsmolekül erhältlich ist durch ein- oder mehrfache konservative Aminosäuresubstitution, wobei die Protease mindestens eine der Aminosäuresubstitutionen P9T/S/H, Q62E, D101E, N130D, G166S, N187H, S216C, N238K oder Q271E an den Positionen, die den Positionen 9, 62, 101, 130, 166, 187, 216, 238 und 271 gemäß SEQ ID NO:2 entsprechen, aufweist.

In weiteren Ausführungsformen ist die Protease dadurch gekennzeichnet, dass sie aus einer erfindungsgemäßen Protease als Ausgangsmolekül erhältlich ist durch Fragmentierung, Deletions-, Insertions- oder Substitutionsmutagenese und eine Aminosäuresequenz umfasst, die über eine Länge von mindestens 50, 60, 70, 80, 90, 100, 110, 120, 130, 140, 150, 160, 170, 180, 190, 200, 210, 220, 230, 240, 250, 260, 265, 270, 271, 272, 273 oder 274 zusammenhängenden Aminosäuren mit dem Ausgangsmolekül übereinstimmt, wobei die Protease mindestens eine der Aminosäuresubstitutionen P9T/S/H, Q62E, D101E, N130D, G166S, N187H, S216C, N238K oder Q271E an den Positionen, die den Positionen 9, 62, 101, 130, 166, 187, 216, 238 und 271 gemäß SEQ ID NO:2 entsprechen, umfasst.

Die weiteren Aminosäurepositionen werden hierbei durch ein Alignment der Aminosäuresequenz einer erfindungsgemäßen Protease mit der Aminosäuresequenz der Protease aus *Bacillus pumilus,* wie sie in SEQ ID NO:2 angegeben ist, definiert. Weiterhin richtet sich die Zuordnung der Positionen nach dem reifen (maturen) Protein. Diese Zuordnung ist insbesondere auch anzuwenden, wenn die Aminosäuresequenz einer erfindungsgemäßen Protease eine höhere Zahl von Aminosäurenresten umfasst als die Protease aus *Bacillus pumilus* gemäß SEQ ID NO:2. Ausgehend von den genannten Positionen in der Aminosäuresequenz der Protease aus *Bacillus pumilus* sind die Veränderungspositionen in einer erfindungsgemäßen Protease diejenigen, die eben diesen Positionen in einem Alignment zugeordnet sind.

Vorteilhafte Positionen für Sequenzveränderungen, insbesondere Substitutionen, der Protease aus *Bacillus pumilus,* die übertragen auf homologe Positionen der erfindungsgemäßen Proteasen bevorzugt von Bedeutung sind und der Protease vorteilhafte funktionelle Eigenschaften verleihen, sind demnach die Positionen, die in einem Alignment den Positionen 9, 62, 101, 130, 166, 187, 216, 238 und 271 in SEQ ID NO:2 entsprechen, d.h. in der Zählung gemäß SEQ ID NO:2. An den genannten Positionen liegen in dem Wildtypmolekül der Protease aus *Bacillus pumilus* folgende Aminosäurereste: P9, Q62, D101, N130, G166, N187, S216, N238 und Q271.

Eine weitere Bestätigung der korrekten Zuordnung der zu verändernden Aminosäuren, d.h. insbesondere deren funktionelle Entsprechung, können Vergleichsversuche liefern, wonach die beiden auf der Basis eines Alignments einander zugeordneten Positionen in beiden miteinander verglichenen Proteasen auf die gleiche Weise verändert werden und beobachtet wird, ob bei beiden die enzymatische Aktivität auf gleiche Weise verändert wird. Geht beispielsweise ein Aminosäureaustausch in einer bestimmten Position der Protease aus *Bacillus pumilus* gemäß SEQ ID NO:2 mit einer Veränderung eines enzymatischen Parameters einher, beispielsweise mit der Erhöhung des K_{M}-Wertes, und wird eine entsprechende Veränderung des enzymatischen Parameters, beispielsweise also ebenfalls eine Erhöhung des K_{M}-Wertes, in einer erfindungsgemäßen Protease-Variante beobachtet, deren Aminosäureaustausch durch dieselbe eingeführte Aminosäure erreicht wurde, so ist hierin eine Bestätigung der korrekten Zuordnung zu sehen.

Alle genannten Sachverhalte sind auch auf die erfindungsgemäßen Verfahren zur Herstellung einer Protease anwendbar. Demnach umfasst ein erfindungsgemäßes Verfahren ferner einen oder mehrere der folgenden Verfahrensschritte:
a) Einbringen einer ein- oder mehrfachen konservativen Aminosäuresubstitution, wobei die Protease mindestens eine der Aminosäuresubstitutionen P9T/S/H, Q62E, D101E, N130D, G166S, N187H, S216C, N238K oder Q271E an den Positionen, die den Positionen 9, 62, 101, 130, 166, 187, 216, 238 und 271 gemäß SEQ ID NO:2 entsprechen, umfasst;
b) Veränderung der Aminosäuresequenz durch Fragmentierung, Deletions-, Insertions- oder Substitutionsmutagenese derart, dass die Protease eine Aminosäuresequenz umfasst, die über eine Länge von mindestens 50, 60, 70, 80, 90, 100, 110, 120, 130, 140, 150, 160, 170, 180, 190, 200, 210, 220, 230, 240, 250, 260, 265, 270, 271, 272, 273 oder 274 zusammenhängenden Aminosäuren mit dem Ausgangsmolekül übereinstimmt, wobei die Protease mindestens eine der Aminosäuresubstitutionen P9T/S/H, Q62E, D101E, N130D, G166S, N187H, S216C, N238K oder Q271E an den Positionen, die den Positionen 9, 62, 101, 130, 166, 187, 216, 238 und 271 gemäß SEQ ID NO:2 entsprechen, umfasst.

Sämtliche Ausführungen gelten auch für die erfindungsgemäßen Verfahren.

In weiteren Ausgestaltungen der Erfindung ist die Protease beziehungsweise die mit einem erfindungsgemäßen Verfahren hergestellte Protease noch mindestens zu 90%, 90,5%, 91%, 91,5%, 92%, 92,5%, 93%, 93,5%, 94%, 94,5%, 95%, 95,5%, 96%, 96,5%, 97%, 97,5%, 98%, 98,5%, oder 98,8% identisch zu der in SEQ ID NO:2 angegebenen Aminosäuresequenz über deren Gesamtlänge. Alternativ ist die Protease beziehungsweise die mit einem erfindungsgemäßen Verfahren hergestellte Protease noch mindestens zu 90%, 90,5%, 91%, 91,5%, 92%, 92,5%, 93%, 93,5%, 94%, 94,5%, 95%, 95,5%, 96%, 96,5%, 97%, 97,5%, oder 98% identisch zu einer der in SEQ ID Nos:3-11 angegebenen Aminosäuresequenzen über deren Gesamtlänge. Die Protease beziehungsweise die mit einem erfindungsgemäßen Verfahren hergestellte Protease weist eine Aminosäuresubstitution an mindestens einer der Positionen P9, Q62, D101, N130, G166, N187, S216, N238 oder Q271, jeweils bezogen auf die Nummerierung gemäß SEQ ID NO:2, auf. In bevorzugteren Ausführungsformen ist die Aminosäuresubstitution mindestens eine ausgewählt aus der Gruppe bestehend aus P9T/S/H, Q62E, D101 E, N130D, G166S, N187H, S216C, N238K und Q271E, jeweils bezogen auf die Nummerierung gemäß SEQ ID NO:2. In weiter bevorzugten Ausführungsformen umfasst die Protease eine der folgenden Aminosäuresubstitutionsvarianten: (i) P9T; (ii) Q271E; (iii) P9S und N238K; (iv) P9H; (v) N187H; (vi) S216C; (vii) Q62E und N130D; (viii) Q62E, D101E, N130D, G166S und Q271E; oder (ix) P9T, N187H, S216C und Q271E.

Ein weiterer Gegenstand der Erfindung ist eine zuvor beschriebene Protease, die zusätzlich stabilisiert ist, insbesondere durch eine oder mehrere Mutationen, beispielsweise Substitutionen, oder durch Kopplung an ein Polymer. Eine Erhöhung der Stabilität bei der Lagerung und/oder während des Einsatzes, beispielsweise beim Waschprozess, führt dazu, dass die enzymatische Aktivität länger anhält und damit die Reinigungsleistung verbessert wird. Grundsätzlich kommen alle im Stand der Technik beschriebenen und/oder zweckmäßigen Stabilisierungsmöglichkeiten in Betracht. Bevorzugt sind solche Stabilisierungen, die über Mutationen des Enzyms selbst erreicht werden, da solche Stabilisierungen im Anschluss an die Gewinnung des Enzyms keine weiteren Arbeitsschritte erfordern. Beispiele für hierfür geeignete Sequenzveränderungen sind vorstehend genannt. Weitere geeignete Sequenzveränderungen sind aus dem Stand der Technik bekannt.

Weitere Möglichkeiten der Stabilisierung sind beispielsweise:
- Veränderung der Bindung von Metallionen, insbesondere der Calcium-Bindungsstellen, beispielsweise durch Austauschen von einer oder mehreren der an der Calcium-Bindung beteiligten Aminosäure(n) gegen eine oder mehrere negativ geladene Aminosäuren und/oder durch Einführen von Sequenzveränderungen in mindestens einer der Folgen der beiden Aminosäuren Arginin/Glycin;
- Schutz gegen den Einfluss von denaturierenden Agentien wie Tensiden durch Mutationen, die eine Veränderung der Aminosäuresequenz auf oder an der Oberfläche des Proteins bewirken;
- Austausch von Aminosäuren, die nahe dem N-Terminus liegen, gegen solche, die vermutlich über nicht-kovalente Wechselwirkungen mit dem Rest des Moleküls in Kontakt treten und somit einen Beitrag zur Aufrechterhaltung der globulären Struktur leisten.

Bevorzugte Ausführungsformen sind solche, bei denen das Enzym auf mehrere Arten stabilisiert wird, da mehrere stabilisierende Mutationen additiv oder synergistisch wirken.

Ein weiterer Gegenstand der Erfindung ist eine Protease wie vorstehend beschrieben, die dadurch gekennzeichnet ist, dass sie mindestens eine chemische Modifikation aufweist. Eine Protease mit einer solchen Veränderung wird als Derivat bezeichnet, d.h. die Protease ist derivatisiert.

Unter Derivaten werden im Sinne der vorliegenden Anmeldung demnach solche Proteine verstanden, deren reine Aminosäurekette chemisch modifiziert worden ist. Solche Derivatisierungen können beispielsweise *in vivo* durch die Wirtszelle erfolgen, die das Protein exprimiert. Diesbezüglich sind Kopplungen niedrigmolekularer Verbindungen wie von Lipiden oder Oligosacchariden besonders hervorzuheben. Derivatisierungen können aber auch *in vitro* durchgeführt werden, etwa durch die chemische Umwandlung einer Seitenkette einer Aminosäure oder durch kovalente Bindung einer anderen Verbindung an das Protein. Beispielsweise ist die Kopplung von Aminen an Carboxylgruppen eines Enzyms zur Veränderung des isoelektrischen Punkts möglich. Eine solche andere Verbindung kann auch ein weiteres Protein sein, das beispielsweise über bifunktionelle chemische Verbindungen an ein erfindungsgemäßes Protein gebunden wird. Ebenso ist unter Derivatisierung die kovalente Bindung an einen makromolekularen Träger zu verstehen, oder auch ein nichtkovalenter Einschluss in geeignete makromolekulare Käfigstrukturen. Derivatisierungen können beispielsweise die Substratspezifität oder die Bindungsstärke an das Substrat beeinflussen oder eine vorübergehende Blockierung der enzymatischen Aktivität herbeiführen, wenn es sich bei der angekoppelten Substanz um einen Inhibitor handelt. Dies kann beispielsweise für den Zeitraum der Lagerung sinnvoll sein. Derartige Modifikationen können ferner die Stabilität oder die enzymatische Aktivität beeinflussen. Sie können ferner auch dazu dienen, die Allergenizität und/oder Immunogenizität des Proteins herabzusetzen und damit beispielsweise dessen Hautverträglichkeit zu erhöhen. Beispielsweise können Kopplungen mit makromolekularen Verbindungen, beispielsweise Polyethylenglykol, das Protein hinsichtlich der Stabilität und/oder Hautverträglichkeit verbessern. Unter Derivaten eines erfindungsgemäßen Proteins können im weitesten Sinne auch Präparationen dieser Proteine verstanden werden. Je nach Gewinnung, Aufarbeitung oder Präparation kann ein Protein mit diversen anderen Stoffen kombiniert sein, beispielsweise aus der Kultur der produzierenden Mikroorganismen. Ein Protein kann auch, beispielsweise zur Erhöhung seiner Lagerstabilität, mit anderen Stoffen gezielt versetzt worden sein. Erfindungsgemäß sind deshalb auch alle Präparationen eines erfindungsgemäßen Proteins. Das ist auch unabhängig davon, ob es in einer bestimmten Präparation tatsächlich diese enzymatische Aktivität entfaltet oder nicht. Denn es kann gewünscht sein, dass es bei der Lagerung keine oder nur geringe Aktivität besitzt, und erst zum Zeitpunkt der Verwendung seine enzymatische Funktion entfaltet. Dies kann beispielsweise über entsprechende Begleitstoffe gesteuert werden. Insbesondere die gemeinsame Präparation von Proteasen mit spezifischen Inhibitoren ist diesbezüglich möglich.

Unter allen vorstehend beschriebenen Proteasen beziehungsweise Proteasevarianten und/oder Derivaten sind im Rahmen der vorliegenden Erfindung diejenigen besonders bevorzugt, deren Stabilität und/oder Aktivität mindestens einer derjenigen der Proteasen gemäß SEQ ID Nos: 3-11 entspricht, und/oder deren Reinigungsleistung mindestens einer derjenigen der Proteasen gemäß SEQ ID Nos: 3-11 entspricht, wobei die Reinigungsleistung in einem Waschsystem bestimmt wird wie vorstehend beschrieben.

Ein weiterer Gegenstand der Erfindung ist eine Nukleinsäure, die für eine erfindungsgemäße Protease kodiert, sowie ein Vektor enthaltend eine solche Nukleinsäure, insbesondere ein Klonierungsvektor oder ein Expressionsvektor. Eine solche Nukleinsäure kann als kodierende Sequenz die in SEQ ID NO:1 angegebene Nukleotidsequenz aufweisen, die an den entsprechenden Positionen mutiert ist, um die gewünschte Aminosäuresubstitution zu ergeben.

Hierbei kann es sich um DNA- oder RNA-Moleküle handeln. Sie können als Einzelstrang, als ein zu diesem Einzelstrang komplementärer Einzelstrang oder als Doppelstrang vorliegen. Insbesondere bei DNA-Molekülen sind die Sequenzen beider komplementärer Stränge in jeweils allen drei möglichen Leserastern zu berücksichtigen. Ferner ist zu berücksichtigen, dass verschiedene Codons, also Basentriplets, für die gleichen Aminosäuren kodieren können, so dass eine bestimmte Aminosäuresequenz von mehreren unterschiedlichen Nukleinsäuren kodiert werden kann. Auf Grund dieser Degeneriertheit des genetischen Codes sind sämtliche Nukleinsäuresequenzen in diesen Erfindungsgegenstand mit eingeschlossen, die eine der vorstehend beschriebenen Proteasen kodieren können. Der Fachmann ist in der Lage, diese Nukleinsäuresequenzen zweifelsfrei zu bestimmen, da trotz der Degeneriertheit des genetischen Codes einzelnen Codons definierte Aminosäuren zuzuordnen sind. Daher kann der Fachmann ausgehend von einer Aminosäuresequenz für diese Aminosäuresequenz kodierende Nukleinsäuren problemlos ermitteln. Weiterhin können bei erfindungsgemäßen Nukleinsäuren ein oder mehrere Codons durch synonyme Codons ersetzt sein. Dieser Aspekt bezieht sich insbesondere auf die heterologe Expression der erfindungsgemäßen Enzyme. So besitzt jeder Organismus, beispielsweise eine Wirtszelle eines Produktionsstammes, eine bestimmte Codon-Verwendung. Unter Codon-Verwendung wird die Übersetzung des genetischen Codes in Aminosäuren durch den jeweiligen Organismus verstanden. Es kann zu Engpässen in der Proteinbiosynthese kommen, wenn die auf der Nukleinsäure liegenden Codons in dem Organismus einer vergleichsweise geringen Zahl von beladenen tRNA-Molekülen gegenüberstehen. Obwohl für die gleiche Aminosäure kodierend führt das dazu, dass in dem Organismus ein Codon weniger effizient translatiert wird als ein synonymes Codon, das für dieselbe Aminosäure kodiert. Auf Grund des Vorliegens einer höheren Anzahl von tRNA-Molekülen für das synonyme Codon kann dieses in dem Organismus effizienter translatiert werden.

Einem Fachmann ist es über heutzutage allgemein bekannte Methoden, wie beispielsweise die chemische Synthese oder die Polymerase-Kettenreaktion (PCR) in Verbindung mit molekularbiologischen und/oder proteinchemischen Standardmethoden möglich, anhand bekannter DNA- und/oder Aminosäuresequenzen die entsprechenden Nukleinsäuren bis hin zu vollständigen Genen herzustellen. Derartige Methoden sind beispielsweise aus Sambrook, J., Fritsch, E.F. and Maniatis, T. 2001. Molecular cloning: a laboratory manual, 3. Edition Cold Spring Laboratory Press. bekannt.

Unter Vektoren werden im Sinne der vorliegenden Erfindung aus Nukleinsäuren bestehende Elemente verstanden, die als kennzeichnenden Nukleinsäurebereich eine erfindungsgemäße Nukleinsäure enthalten. Sie vermögen diese in einer Spezies oder einer Zellinie über mehrere Generationen oder Zellteilungen hinweg als stabiles genetisches Element zu etablieren. Vektoren sind insbesondere bei der Verwendung in Bakterien spezielle Plasmide, also zirkulare genetische Elemente. Im Rahmen der vorliegenden Erfindung wird eine erfindungsgemäße Nukleinsäure in einen Vektor kloniert. Zu den Vektoren zählen beispielsweise solche, deren Ursprung bakterielle Plasmide, Viren oder Bacteriophagen sind, oder überwiegend synthetische Vektoren oder Plasmide mit Elementen verschiedenster Herkunft. Mit den weiteren jeweils vorhandenen genetischen Elementen vermögen Vektoren sich in den betreffenden Wirtszellen über mehrere Generationen hinweg als stabile Einheiten zu etablieren. Sie können extrachromosomal als eigene Einheiten vorliegen oder in ein Chromosom oder chromosomale DNA integrieren.

Expressionsvektoren umfassen Nukleinsäuresequenzen, die sie dazu befähigen, in den sie enthaltenden Wirtszellen, vorzugsweise Mikroorganismen, besonders bevorzugt Bakterien, zu replizieren und dort eine enthaltene Nukleinsäure zur Expression zu bringen. Die Expression wird insbesondere von dem oder den Promotoren beeinflusst, welche die Transkription regulieren. Prinzipiell kann die Expression durch den natürlichen, ursprünglich vor der zu exprimierenden Nukleinsäure lokalisierten Promotor erfolgen, aber auch durch einen auf dem Expressionsvektor bereitgestellten Promotor der Wirtszelle oder auch durch einen modifizierten oder einen völlig anderen Promotor eines anderen Organismus oder einer anderen Wirtszelle. Im vorliegenden Fall wird zumindest ein Promotor für die Expression einer erfindungsgemäßen Nukleinsäure zur Verfügung gestellt und für deren Expression genutzt. Expressionsvektoren können ferner regulierbar sein, beispielsweise durch Änderung der Kultivierungsbedingungen oder bei Erreichen einer bestimmten Zelldichte der sie enthaltenen Wirtszellen oder durch Zugabe von bestimmten Substanzen, insbesondere Aktivatoren der Genexpression. Ein Beispiel für eine solche Substanz ist das Galactose-Derivat Isopropyl-β-D-thiogalactopyranosid (IPTG), welches als Aktivator des bakteriellen Lactose-Operons (lac-Operons) verwendet wird. Im Gegensatz zu Expressionsvektoren wird die enthaltene Nukleinsäure in Klonierungsvektoren nicht exprimiert.

Ein weiterer Gegenstand der Erfindung ist eine nicht menschliche Wirtszelle, die eine erfindungsgemäße Nukleinsäure oder einen erfindungsgemäßen Vektor beinhaltet, oder die eine erfindungsgemäße Protease beinhaltet, insbesondere eine, die die Protease in das die Wirtszelle umgebende Medium sezerniert. Bevorzugt wird eine erfindungsgemäße Nukleinsäure oder ein erfindungsgemäßer Vektor in einen Mikroorganismus transformiert, der dann eine erfindungsgemäße Wirtszelle darstellt. Alternativ können auch einzelne Komponenten, d.h. Nukleinsäure-Teile oder -Fragmente einer erfindungsgemäßen Nukleinsäure derart in eine Wirtszelle eingebracht werden, dass die dann resultierende Wirtszelle eine erfindungsgemäße Nukleinsäure oder einen erfindungsgemäßen Vektor enthält. Dieses Vorgehen eignet sich besonders dann, wenn die Wirtszelle bereits einen oder mehrere Bestandteile einer erfindungsgemäßen Nukleinsäure oder eines erfindungsgemäßen Vektors enthält und die weiteren Bestandteile dann entsprechend ergänzt werden. Verfahren zur Transformation von Zellen sind im Stand der Technik etabliert und dem Fachmann hinlänglich bekannt. Als Wirtszellen eignen sich prinzipiell alle Zellen, das heißt prokaryotische oder eukaryotische Zellen. Bevorzugt sind solche Wirtszellen, die sich genetisch vorteilhaft handhaben lassen, was beispielsweise die Transformation mit der Nukleinsäure oder dem Vektor und dessen stabile Etablierung angeht, beispielsweise einzellige Pilze oder Bakterien. Ferner zeichnen sich bevorzugte Wirtszellen durch eine gute mikrobiologische und biotechnologische Handhabbarkeit aus. Das betrifft beispielsweise leichte Kultivierbarkeit, hohe Wachstumsraten, geringe Anforderungen an Fermentationsmedien und gute Produktions- und Sekretionsraten für Fremdproteine. Bevorzugte erfindungsgemäße Wirtszellen sezernieren das (transgen) exprimierte Protein in das die Wirtszellen umgebende Medium. Ferner können die Proteasen von den sie produzierenden Zellen nach deren Herstellung modifiziert werden, beispielsweise durch Anknüpfung von Zuckermolekülen, Formylierungen, Aminierungen, usw. Solche posttranslationale Modifikationen können die Protease funktionell beeinflussen.

Weitere bevorzugte Ausführungsformen stellen solche Wirtszellen dar, die aufgrund genetischer Regulationselemente, die beispielsweise auf dem Vektor zur Verfügung gestellt werden, aber auch von vornherein in diesen Zellen vorhanden sein können, in ihrer Aktivität regulierbar sind. Beispielsweise durch kontrollierte Zugabe von chemischen Verbindungen, die als Aktivatoren dienen, durch Änderung der Kultivierungsbedingungen oder bei Erreichen einer bestimmten Zelldichte können diese zur Expression angeregt werden. Dies ermöglicht eine wirtschaftliche Produktion der erfindungsgemäßen Proteine. Ein Beispiel für eine solche Verbindung ist IPTG wie vorstehend beschrieben.

Bevorzugte Wirtszellen sind prokaryontische oder bakterielle Zellen. Bakterien zeichnen sich durch kurze Generationszeiten und geringe Ansprüche an die Kultivierungsbedingungen aus. Dadurch können kostengünstige Kultivierungsverfahren oder Herstellungsverfahren etabliert werden. Zudem verfügt der Fachmann bei Bakterien in der Fermentationstechnik über einen reichhaltigen Erfahrungsschatz. Für eine spezielle Produktion können aus verschiedensten, im Einzelfall experimentell zu ermittelnden Gründen wie Nährstoffquellen, Produktbildungsrate, Zeitbedarf usw., gramnegative oder grampositive Bakterien geeignet sein.

Bei gramnegativen Bakterien wie beispielsweise *Escherichia coli* wird eine Vielzahl von Proteinen in den periplasmatischen Raum sezerniert, also in das Kompartiment zwischen den beiden die Zellen einschließenden Membranen. Dies kann für spezielle Anwendungen vorteilhaft sein. Ferner können auch gramnegative Bakterien so ausgestaltet werden, dass sie die exprimierten Proteine nicht nur in den periplasmatischen Raum, sondern in das das Bakterium umgebende Medium ausschleusen. Grampositive Bakterien wie beispielsweise Bacilli oder Actinomyceten oder andere Vertreter der Actinomycetales besitzen demgegenüber keine äußere Membran, so dass sezernierte Proteine sogleich in das die Bakterien umgebende Medium, in der Regel das Nährmedium, abgegeben werden, aus welchem sich die exprimierten Proteine aufreinigen lassen. Sie können aus dem Medium direkt isoliert oder weiter prozessiert werden. Zudem sind grampositive Bakterien mit den meisten Herkunftsorganismen für technisch wichtige Enzyme verwandt oder identisch und bilden meist selbst vergleichbare Enzyme, so dass sie über eine ähnliche Codon-Verwendung verfügen und ihr Protein-Syntheseapparat naturgemäß entsprechend ausgerichtet ist.

Erfindungsgemäße Wirtszellen können hinsichtlich ihrer Anforderungen an die Kulturbedingungen verändert sein, andere oder zusätzliche Selektionsmarker aufweisen oder noch andere oder zusätzliche Proteine exprimieren. Es kann sich insbesondere auch um solche Wirtszellen handeln, die mehrere Proteine oder Enzyme transgen exprimieren.

Die vorliegende Erfindung ist prinzipiell auf alle Mikroorganismen, insbesondere auf alle fermentierbaren Mikroorganismen, besonders bevorzugt auf solche der Gattung *Bacillus,* anwendbar und führt dazu, dass sich durch den Einsatz solcher Mikroorganismen erfindungsgemäße Proteine herstellen lassen. Solche Mikroorganismen stellen dann Wirtszellen im Sinne der Erfindung dar.

In einer weiteren Ausführungsform der Erfindung ist die Wirtszelle dadurch gekennzeichnet, dass sie ein Bakterium ist, bevorzugt eines, das ausgewählt ist aus der Gruppe der Gattungen von E*scherichia, Klebsiella, Bacillus, Staphylococcus, Corynebakterium, Arthrobacter, Streptomyces, Stenotrophomonas* und *Pseudomonas,* weiter bevorzugt eines, das ausgewählt ist aus der Gruppe von *Escherichia coli, Klebsiella planticola, Bacillus licheniformis, Bacillus lentus, Bacillus amyloliquefaciens, Bacillus subtilis, Bacillus alcalophilus, Bacillus globigii, Bacillus gibsonii, Bacillus clausii, Bacillus halodurans, Bacillus pumilus, Staphylococcus carnosus, Corynebacterium glutamicum, Arthrobacter oxidans, Streptomyces lividans, Streptomyces coelicolor* und *Stenotrophomonas maltophilia.*

Die Wirtszelle kann aber auch eine eukaryontische Zelle sein, die dadurch gekennzeichnet ist, dass sie einen Zellkern besitzt. Einen weiteren Gegenstand der Erfindung stellt daher eine Wirtszelle dar, die dadurch gekennzeichnet ist, dass sie einen Zellkern besitzt. Im Gegensatz zu prokaryontischen Zellen sind eukaryontische Zellen in der Lage, das gebildete Protein posttranslational zu modifizieren. Beispiele dafür sind Pilze wie Actinomyceten oder Hefen wie *Saccharomyces* oder *Kluyveromyces.* Dies kann beispielsweise dann besonders vorteilhaft sein, wenn die Proteine im Zusammenhang mit ihrer Synthese spezifische Modifikationen erfahren sollen, die derartige Systeme ermöglichen. Zu den Modifikationen, die eukaryontische Systeme besonders im Zusammenhang mit der Proteinsynthese durchführen, gehören beispielsweise die Bindung niedermolekularer Verbindungen wie Membrananker oder Oligosaccharide. Derartige Oligosaccharid-Modifikationen können beispielsweise zur Senkung der Allergenizität eines exprimierten Proteins wünschenswert sein. Auch eine Coexpression mit den natürlicherweise von derartigen Zellen gebildeten Enzymen, wie beispielsweise Cellulasen, kann vorteilhaft sein. Ferner können sich beispielsweise thermophile pilzliche Expressionssysteme besonders zur Expression temperaturbeständiger Proteine oder Varianten eignen.

Die erfindungsgemäßen Wirtszellen werden in üblicher Weise kultiviert und fermentiert, beispielsweise in diskontinuierlichen oder kontinuierlichen Systemen. Im ersten Fall wird ein geeignetes Nährmedium mit den Wirtszellen beimpft und das Produkt nach einem experimentell zu ermittelnden Zeitraum aus dem Medium geerntet. Kontinuierliche Fermentationen zeichnen sich durch Erreichen eines Fließgleichgewichts aus, in dem über einen vergleichsweise langen Zeitraum Zellen teilweise absterben aber auch nachwachsen und gleichzeitig aus dem Medium das gebildete Protein entnommen werden kann.

Erfindungsgemäße Wirtszellen werden bevorzugt verwendet, um erfindungsgemäße Proteasen herzustellen. Ein weiterer Gegenstand der Erfindung ist daher ein Verfahren zur Herstellung einer Protease umfassend
a) Kultivieren einer erfindungsgemäßen Wirtszelle, und
b) Isolieren der Protease aus dem Kulturmedium oder aus der Wirtszelle.

Dieser Erfindungsgegenstand umfasst bevorzugt Fermentationsverfahren. Fermentationsverfahren sind an sich aus dem Stand der Technik bekannt und stellen den eigentlichen großtechnischen Produktionsschritt dar, in der Regel gefolgt von einer geeigneten Aufreinigungsmethode des hergestellten Produktes, beispielsweise der erfindungsgemäßen Proteasen. Alle Fermentationsverfahren, die auf einem entsprechenden Verfahren zur Herstellung einer erfindungsgemäßen Protease beruhen, stellen Ausführungsformen dieses Erfindungsgegenstandes dar.

Fermentationsverfahren, die dadurch gekennzeichnet sind, dass die Fermentation über eine Zulaufstrategie durchgeführt wird, kommen insbesondere in Betracht. Hierbei werden die Medienbestandteile, die durch die fortlaufende Kultivierung verbraucht werden, zugefüttert. Hierdurch können beträchtliche Steigerungen sowohl in der Zelldichte als auch in der Zellmasse beziehungsweise Trockenmasse und/oder insbesondere in der Aktivität der interessierenden Protease erreicht werden. Ferner kann die Fermentation auch so gestaltet werden, dass unerwünschte Stoffwechselprodukte herausgefiltert oder durch Zugabe von Puffer oder jeweils passende Gegenionen neutralisiert werden.

Die hergestellte Protease kann aus dem Fermentationsmedium geerntet werden. Ein solches Fermentationsverfahren ist gegenüber einer Isolation der Protease aus der Wirtszelle, d.h. einer Produktaufbereitung aus der Zellmasse (Trockenmasse) bevorzugt, erfordert jedoch die Zurverfügungstellung von geeigneten Wirtszellen oder von einem oder mehreren geeigneten Sekretionsmarkern oder -mechanismen und/oder Transportsystemen, damit die Wirtszellen die Protease in das Fermentationsmedium sezernieren. Ohne Sekretion kann alternativ die Isolation der Protease aus der Wirtszelle, d.h. eine Aufreinigung derselben aus der Zellmasse, erfolgen, beispielsweise durch Fällung mit Ammoniumsulfat oder Ethanol, oder durch chromatographische Reinigung.

Alle vorstehend ausgeführten Sachverhalte können zu Verfahren kombiniert werden, um erfindungsgemäße Protease herzustellen.

Ein weiterer Gegenstand der Erfindung ist ein Mittel, das dadurch gekennzeichnet ist, dass es eine erfindungsgemäße Protease wie vorstehend beschrieben enthält. Bevorzugt ist das Mittel als ein Wasch- oder Reinigungsmittel.

Zu diesem Erfindungsgegenstand zählen alle denkbaren Wasch- oder Reinigungsmittelarten, sowohl Konzentrate als auch unverdünnt anzuwendende Mittel, zum Einsatz im kommerziellen Maßstab, in der Waschmaschine oder bei der Handwäsche beziehungsweise -reinigung. Dazu gehören beispielsweise Waschmittel für Textilien, Teppiche, oder Naturfasern, für die die Bezeichnung Waschmittel verwendet wird. Dazu gehören beispielsweise auch Geschirrspülmittel für Geschirrspülmaschinen oder manuelle Geschirrspülmittel oder Reiniger für harte Oberflächen wie Metall, Glas, Porzellan, Keramik, Kacheln, Stein, lackierte Oberflächen, Kunststoffe, Holz oder Leder, für die die Bezeichnung Reinigungsmittel verwendet wird, also neben manuellen und maschinellen Geschirrspülmitteln beispielsweise auch Scheuermittel, Glasreiniger, WC-Duftspüler, usw. Zu den Wasch- und Reinigungsmittel im Rahmen der Erfindung zählen ferner Waschhilfsmittel, die bei der manuellen oder maschinellen Textilwäsche zum eigentlichen Waschmittel hinzudosiert werden, um eine weitere Wirkung zu erzielen. Ferner zählen zu Wasch- und Reinigungsmittel im Rahmen der Erfindung auch Textilvor- und Nachbehandlungsmittel, also solche Mittel, mit denen das Wäschestück vor der eigentlichen Wäsche in Kontakt gebracht wird, beispielsweise zum Anlösen hartnäckiger Verschmutzungen, und auch solche Mittel, die in einem der eigentlichen Textilwäsche nachgeschalteten Schritt dem Waschgut weitere wünschenswerte Eigenschaften wie angenehmen Griff, Knitterfreiheit oder geringe statische Aufladung verleihen. Zu letztgenannten Mittel werden u.a. die Weichspüler gerechnet.

Die erfindungsgemäßen Wasch- oder Reinigungsmittel, die als pulverförmige Feststoffe, in nachverdichteter Teilchenform, als homogene Lösungen oder Suspensionen vorliegen können, können neben einer erfindungsgemäßen Protease alle bekannten und in derartigen Mitteln üblichen Inhaltsstoffe enthalten, wobei bevorzugt mindestens ein weiterer Inhaltsstoff in dem Mittel vorhanden ist. Die erfindungsgemäßen Mittel können insbesondere Tenside, Builder (Gerüststoffe), Persauerstoffverbindungen oder Bleichaktivatoren enthalten. Ferner können sie wassermischbare organische Lösungsmittel, weitere Enzyme, Sequestrierungsmittel, Elektrolyte, pH-Regulatoren und/oder weitere Hilfsstoffe wie optische Aufheller, Vergrauungsinhibitoren, Schaumregulatoren sowie Farb- und Duftstoffe sowie Kombinationen hiervon enthalten.

Insbesondere eine Kombination einer erfindungsgemäßen Protease mit einem oder mehreren weiteren Inhaltsstoff(en) des Mittels ist vorteilhaft, da ein solches Mittel in bevorzugten erfindungsgemäßen Ausgestaltungen eine verbesserte Reinigungsleistung durch sich ergebende Synergismen aufweist. Insbesondere durch die Kombination einer erfindungsgemäßen Protease mit einem Tensid und/oder einem Builder (Gerüststoff) und/oder einer Persauerstoffverbindung und/oder einem Bleichaktivator kann ein solcher Synergismus erreicht werden. Da die hierin beschriebenen Protease bereits ohne Enzymstabilisator, wie beispielsweise Borsäure, eine hohe Enzymstabilität aufweisen, kann in verschiedenen Ausführungsformen auf den Zusatz solcher Stabilisatoren zu den enzymhaltigen Mitteln verzichtet bzw. die Menge dieser Stabilisatoren verringert werden.

Vorteilhafte Inhaltsstoffe erfindungsgemäßer Mittel sind offenbart in der internationalen Patentanmeldung WO2009/121725, dort beginnend auf Seite 5, vorletzter Absatz, und endend auf Seite 13 nach dem zweiten Absatz. Auf diese Offenbarung wird ausdrücklich Bezug genommen und der dortige Offenbarungsgehalt in die vorliegende Patentanmeldung einbezogen.

Ein erfindungsgemäßes Mittel enthält die Protease vorteilhafterweise in einer Menge von 2µg bis 20mg, vorzugsweise von 5µg bis 17,5mg, besonders bevorzugt von 20µg bis 15mg und ganz besonders bevorzugt von 50µg bis 10mg pro g des Mittels. Ferner kann die in dem Mittel enthaltene Protease, und/oder weitere Inhaltsstoffe des Mittels, mit einer bei Raumtemperatur oder bei Abwesenheit von Wasser für das Enzym undurchlässigen Substanz umhüllt sein, welche unter Anwendungsbedingungen des Mittels durchlässig für das Enzym wird. Eine solche Ausführungsform der Erfindung ist somit dadurch gekennzeichnet, dass die Protease mit einer bei Raumtemperatur oder bei Abwesenheit von Wasser für die Protease undurchlässigen Substanz umhüllt ist. Weiterhin kann auch das Wasch- oder Reinigungsmittel selbst in einem Behältnis, vorzugsweise einem luftdurchlässigen Behältnis, verpackt sein, aus dem es kurz vor Gebrauch oder während des Waschvorgangs freigesetzt wird.

In weiteren Ausführungsformen der Erfindung ist das Mittel dadurch gekennzeichnet, dass es
(a) in fester Form vorliegt, insbesondere als rieselfähiges Pulver mit einem Schüttgewicht von 300 g/l bis 1200 g/l, insbesondere 500 g/l bis 900 g/l, oder
(b) in pastöser oder in flüssiger Form vorliegt, und/oder
(c) in gelförmiger oder dosierbeutelförmiger (Pouches) Form vorliegt, und/oder
(d) als Einkomponentensystem vorliegt, oder
(e) in mehrere Komponenten aufgeteilt ist.

Diese Ausführungsformen der vorliegenden Erfindung umfassen alle festen, pulverförmigen, flüssigen, gelförmigen oder pastösen Darreichungsformen erfindungsgemäßer Mittel, die gegebenenfalls auch aus mehreren Phasen bestehen können sowie in komprimierter oder nicht komprimierter Form vorliegen können. Das Mittel kann als rieselfähiges Pulver vorliegen, insbesondere mit einem Schüttgewicht von 300 g/l bis 1200 g/l, insbesondere 500 g/l bis 900 g/l oder 600 g/l bis 850 g/l. Zu den festen Darreichungsformen des Mittels zählen ferner Extrudate, Granulate, Tabletten oder Pouches. Alternativ kann das Mittel auch flüssig, gelförmig oder pastös sein, beispielsweise in Form eines nicht-wässrigen Flüssigwaschmittels oder einer nicht-wässrigen Paste oder in Form eines wässrigen Flüssigwaschmittels oder einer wasserhaltigen Paste. Weiterhin kann das Mittel als Einkomponentensystem vorliegen. Solche Mittel bestehen aus einer Phase. Alternativ kann ein Mittel auch aus mehreren Phasen bestehen. Ein solches Mittel ist demnach in mehrere Komponenten aufgeteilt.

Erfindungsgemäße Wasch- oder Reinigungsmittel können ausschließlich eine Protease enthalten. Alternativ können sie auch weitere hydrolytische Enzyme oder andere Enzyme in einer für die Wirksamkeit des Mittels zweckmäßigen Konzentration enthalten. Eine weitere Ausführungsform der Erfindung stellen somit Mittel dar, die ferner eines oder mehrere weitere Enzyme umfassen. Als weitere Enzyme bevorzugt einsetzbar sind alle Enzyme, die in dem erfindungsgemäßen Mittel eine katalytische Aktivität entfalten können, insbesondere eine Lipase, Amylase, Cellulase, Hemicellulase, Mannanase, Tannase, Xylanase, Xanthanase, Xyloglucanase, β-Glucosidase, Pektinase, Carrageenase, Perhydrolase, Oxidase, Oxidoreduktase oder andere - von den erfindungsgemäßen Protease unterscheidbare - Protease, sowie deren Gemische. Weitere Enzyme sind in dem Mittel vorteilhafterweise jeweils in einer Menge von 1 x 10⁻⁸ bis 5 Gewichts-Prozent bezogen auf aktives Protein enthalten. Zunehmend bevorzugt ist jedes weitere Enzym in einer Menge von 1 x 10⁻⁷-3 Gew.-%, von 0,00001-1 Gew.-%, von 0,00005-0,5 Gew.-%, von 0,0001 bis 0,1 Gew.-% und besonders bevorzugt von 0,0001 bis 0,05 Gew.-% in erfindungsgemäßen Mitteln enthalten, bezogen auf aktives Protein. Besonders bevorzugt zeigen die Enzyme synergistische Reinigungsleistungen gegenüber bestimmten Anschmutzungen oder Flecken, d.h. die in der Mittelzusammensetzung enthaltenen Enzyme unterstützen sich in ihrer Reinigungsleistung gegenseitig. Ganz besonders bevorzugt liegt ein solcher Synergismus vor zwischen der erfindungsgemäß enthaltenen Protease und einem weiteren Enzym eines erfindungsgemäßen Mittels, darunter insbesondere zwischen der genannten Protease und einer Amylase und/oder einer Lipase und/oder einer Mannanase und/oder einer Cellulase und/oder einer Pektinase. Synergistische Effekte können nicht nur zwischen verschiedenen Enzymen, sondern auch zwischen einem oder mehreren Enzymen und weiteren Inhaltsstoffen des erfindungsgemäßen Mittels auftreten.

Ein weiterer Erfindungsgegenstand ist ein Verfahren zur Reinigung von Textilien oder harten Oberflächen, das dadurch gekennzeichnet ist, dass in mindestens einem Verfahrensschritt ein erfindungsgemäßes Mittel angewendet wird, oder dass in mindestens einem Verfahrensschritt eine erfindungsgemäße Protease katalytisch aktiv wird, insbesondere derart, dass die Protease in einer Menge von 40µg bis 4g, vorzugsweise von 50µg bis 3g, besonders bevorzugt von 100µg bis 2g und ganz besonders bevorzugt von 200µg bis 1g eingesetzt wird.

In verschieden Ausführungsformen zeichnet sich das oben beschriebene Verfahren dadurch aus, dass die Protease bei einer Temperatur von 0-100°C, bevorzugt 0-60°C, weiter bevorzugt 20-40°C und am meisten bevorzugt bei 35°C eingesetzt wird.

Hierunter fallen sowohl manuelle als auch maschinelle Verfahren, wobei maschinelle Verfahren bevorzugt sind. Verfahren zur Reinigung von Textilien zeichnen sich im allgemeinen dadurch aus, dass in mehreren Verfahrensschritten verschiedene reinigungsaktive Substanzen auf das Reinigungsgut aufgebracht und nach der Einwirkzeit abgewaschen werden, oder dass das Reinigungsgut in sonstiger Weise mit einem Waschmittel oder einer Lösung oder Verdünnung dieses Mittels behandelt wird. Entsprechendes gilt für Verfahren zur Reinigung von allen anderen Materialien als Textilien, insbesondere von harten Oberflächen. Alle denkbaren Wasch- oder Reinigungsverfahren können in wenigstens einem der Verfahrensschritte um die Anwendung eines erfindungsgemäßen Wasch- oder Reinigungsmittels oder einer erfindungsgemäßen Protease bereichert werden und stellen dann Ausführungsformen der vorliegenden Erfindung dar. Alle Sachverhalte, Gegenstände und Ausführungsformen, die für erfindungsgemäße Protease und sie enthaltende Mittel beschrieben sind, sind auch auf diesen Erfindungsgegenstand anwendbar. Daher wird an dieser Stelle ausdrücklich auf die Offenbarung an entsprechender Stelle verwiesen mit dem Hinweis, dass diese Offenbarung auch für die vorstehenden erfindungsgemäßen Verfahren gilt.

Da erfindungsgemäße Proteasen natürlicherweise bereits eine hydrolytische Aktivität besitzen und diese auch in Medien entfalten, die sonst keine Reinigungskraft besitzen wie beispielsweise in bloßem Puffer, kann ein einzelner und/oder der einzige Schritt eines solchen Verfahrens darin bestehen, dass als einzige reinigungsaktive Komponente eine erfindungsgemäße Protease mit der Anschmutzung in Kontakt gebracht wird, bevorzugt in einer Pufferlösung oder in Wasser. Dies stellt eine weitere Ausführungsform dieses Erfindungsgegenstandes dar.

Alternative Ausführungsformen dieses Erfindungsgegenstandes stellen auch Verfahren zur Behandlung von Textilrohstoffen oder zur Textilpflege dar, bei denen in wenigstens einem Verfahrensschritt eine erfindungsgemäße Protease aktiv wird. Hierunter sind Verfahren für Textilrohstoffe, Fasern oder Textilien mit natürlichen Bestandteilen bevorzugt, und ganz besonders für solche mit Wolle oder Seide.

Schließlich erfasst die Erfindung auch die Verwendung der hierin beschriebenen Proteasen in Wasch- oder Reinigungsmitteln, beispielsweise wie oben beschrieben, zur (verbesserten) Entfernung von proteinhaltigen Anschmutzungen, beispielsweise von Textilien oder harten Oberflächen.

Alle Sachverhalte, Gegenstände und Ausführungsformen, die für erfindungsgemäße Protease und sie enthaltende Mittel beschrieben sind, sind auch auf diesen Erfindungsgegenstand anwendbar. Daher wird an dieser Stelle ausdrücklich auf die Offenbarung an entsprechender Stelle verwiesen mit dem Hinweis, dass diese Offenbarung auch für die vorstehende erfindungsgemäße Verwendung gilt.

### Beispiele

Alle molekularbiologischen Arbeitsschritte folgen Standardmethoden, wie sie beispielsweise in dem Handbuch von Fritsch, Sambrook und Maniatis "Molecular cloning: a laboratory manual", Cold Spring Harbour Laboratory Press, New York, 1989, oder vergleichbaren einschlägigen Werken angegeben sind. Enzyme und Baukästen (Kits) wurden nach den Angaben der jeweiligen Hersteller eingesetzt.

**Übersicht über die Mutationen:**

| **Variante** | **Sequenz** | | | | | **SEQ ID NO:** |
|---|---|---|---|---|---|---|
| Variante 1 | P9T | | | | | 3 |
| Variante 2 | Q271E | | | | | 4 |
| Variante 3 | P9S | N238K | | | | 5 |
| Variante 4 | P9H | | | | | 6 |
| Variante 5 | N187H | | | | | 7 |
| Variante 6 | S216C | | | | | 8 |
| Variante 7 | Q62E | N130D | | | | 9 |
| Variante 8 | Q62E | D101E | N130D | G166S | Q271E | 10 |
| Variante 9 | P9T | N187H | S216C | Q271E | | 11 |

### Beispiel 1: Lagerstabilität der Enzyme in Waschmittel

Die Proteasen werden auf gleichem Aktivitätslevel in eine Waschmittelmatrix eingerührt und bei 30°C gelagert. Mittels eines üblichen Aktivitätsassays für Proteasen (Hydrolyse von suc-AAPFpNA), wird die Startaktivität und die Restaktivität der Protease nach 42 h und 9 Tagen Lagerung bei 30°C gemessen.

Um harsche Bedingungen zu generieren werden die Proteasen einmal in Waschmittelmatrix ohne Stabilisator (Borsäure) und zum Vergleich einmal mit Borsäure gelagert.

Dies ist die Waschmittelmatrix, die hier verwendet wurde (LSPA+):

| **Chemischer Name** | Gew.-% Aktivsubstanz im Rohmaterial | Gew.-% Aktivsubstanz in der Formulierung |
|---|---|---|
| Wasser demin. | 100 | Rest |
| Alkylbenzolsulfonsäure | 96 | 4,4 |
| Anionische Tenside | 70 | 5,6 |
| C12-C18 Fettsäure Na-Salz | 30 | 2,4 |
| Nicht-ionische Tenside | 100 | 4,4 |
| Phosphonate | 40 | 0,2 |
| Zitronensäure | 100 | 1,4 |
| NaOH | 50 | 0,95 |
| Entschäumer | t.q. | 0,01 |
| Glycerin | 100 | 2 |
| Konservierungsmittel | 100 | 0,08 |
| Ethanol | 93 | 1 |
| Ohne opt. Aufheller, Parfüm, Farbstoff und Enzyme. | | |

| | | |
|---|---|---|
| Diese Matrix wird für die Messungen mit Stabilisator mit 1% Borsäure versehen. | | |

### Beispiel 2: Enzyme

Die Proteasen liegen in Schüttelkolben generierten Überständen in *Bacillus subtilis* vor. Sie werden auf ein gleiches Aktivitätslevel verdünnt. Es werden 50% Waschmittelmatrix +/- Borsäure mit 50% entsprechend verdünnten *Bacillus subtilis* Protease-Überstand versetzt und gut durchmischt. Die verschlossenen Gläser werden bei 30°C inkubiert. Zum Zeitpunkt der Probenahme wird eine gewisse Menge Matrix/Proteasegemisch entnommen und für 20 min bei RT im Probenpuffer (0,1 M Tris/HCI, pH 8,6) durch Rühren gelöst. Dann wird der AAPF Assay wie unten beschrieben durchgeführt.

9 Mutanten (SEQ ID Nos. 3-11) haben sich als vorteilhaft herausgestellt, die Aktivität ist in % vom Anfangswert dargestellt. Dieser wurde direkt nach Zusammenmischen der Proteasen mit der Matrix gemessen und wird jeweils auf 100% gesetzt:

| Protease | Startakt. | 4 d - B(OH)₃ | 4 d + B(OH)₃ | 12 d - B(OH)₃ | 12 d + B(OH)₃ |
|---|---|---|---|---|---|
| SEQ ID NO:2 | 100% | 78% | 106% | 38% | 88% |
| SEQ ID NO:3 | 100% | 99% | 103% | 77% | 92% |
| SEQ ID NO:4 | 100% | 91% | 103% | 56% | 94% |
| SEQ ID NO:5 | 100% | 98% | 105% | 83% | 90% |
| SEQ ID NO:6 | 100% | 102% | 100% | 82% | 92% |
| SEQ ID NO:7 | 100% | 96% | 111% | 63% | 95% |
| SEQ ID NO:8 | 100% | 99% | 106% | 73% | 97% |

Bei den Mutanten gemäß SEQ ID Nos. 9-11 handelt es sich um Rekombinanten, die in einem anderen Versuch gemessen wurden:

| Protease | Startakt. | 3,5 d - B(OH)₃ | 3,5 d + B(OH)₃ |
|---|---|---|---|
| SEQ ID NO:2 | 100% | 76% | 98% |
| SEQ ID NO:9 | 100% | 93% | 110% |
| SEQ ID NO:10 | 100% | 88% | 112% |
| SEQ ID NO:11 | 100% | 93% | 101% |

Alle Mutanten zeigen eine vergleichbare Waschleistung wie der Wildtyp, d.h. sie sind maximal 10% schlechter in der Waschleistung, was im Rahmen der Messschwankung liegt.(Ergebnisse nicht gezeigt).

### Beispiel 3: Protease-Aktivitätsassays

Die Aktivität der Protease wird durch die Freisetzung des Chromophors para-Nitroanilin aus dem Substrat Succinyl Alanin-Alanin-Prolin-Phenylalanin-para-Nitroanilid (AAPFpNA; Bachem L-1400) bestimmt. Die Freisetzung des pNA verursacht eine Zunahme der Extinktion bei 410 nm, deren zeitlicher Verlauf ein Maß für die enzymatische Aktivität ist.

Die Messung erfolgte bei einer Temperatur von 25°C, bei pH 8,6 und einer Wellenlänge von 410 nm. Die Messzeit betrug 5 min bei einem Messintervall von 20 bis 60 Sekunden.
Messansatz:
10 µL AAPF-Lösung (70 mg/mL)
1000 µL Tris/HCI (0,1 M; pH 8,6 mit 0,1% Brij 35)
10 µL verdünnte Proteaselösung
Kinetik erstellt über 5 min bei 25°C (410 nm)

## Patentansprüche

1. Protease umfassend eine Aminosäuresequenz, die über ihre Gesamtlänge mindestens 90% Sequenzidentität mit der in SEQ ID NO:2 angegebenen Aminosäuresequenz aufweist und die eine Aminosäuresubstitution an mindestens einer der Positionen P9, Q62, D101, N130, G166, N187, S216, N238 oder Q271, jeweils bezogen auf die Nummerierung gemäß SEQ ID NO:2, aufweist, wobei die mindestens eine Aminosäuresubstitution aus der Gruppe bestehend aus P9T/S/H, Q62E, D101E, N130D, G166S, N187H, S216C, N238K oder Q271E, jeweils bezogen auf die Nummerierung gemäß SEQ ID NO:2, ausgewählt ist.

2. Protease nach Anspruch 1, wobei die Protease eine der folgenden Aminosäuresubstitutionsvarianten aufweist:
(i) P9T;
(ii) Q271E;
(iii) P9S und N238K;
(iv) P9H;
(v) N187H;
(vi) S216C;
(vii) Q62E und N130D;
(viii) Q62E, D101 E, N130D, G166S und Q271E; oder
(ix) P9T, N187H, S216C und Q271E.

3. Protease, **dadurch gekennzeichnet, dass**
(a) sie aus einer Protease nach Anspruch 1-2 als Ausgangsmolekül erhältlich ist durch ein- oder mehrfache konservative Aminosäuresubstitution, wobei die Protease mindestens eine der Aminosäuresubstitutionen P9T/S/H, Q62E, D101E, N130D, G166S, N187H, S216C, N238K oder Q271E an den Positionen, die den Positionen 9, 62, 101, 130, 166, 187, 216, 238 und 271 gemäß SEQ ID NO:2 entsprechen, aufweist; und/oder
(b) sie aus einer Protease nach Anspruch 1-2 als Ausgangsmolekül erhältlich ist durch Fragmentierung, Deletions-, Insertions- oder Substitutionsmutagenese und eine Aminosäuresequenz umfasst, die über eine Länge von mindestens 50, 60, 70, 80, 90, 100, 110, 120, 130, 140, 150, 160, 170, 180, 190, 200, 210, 220, 230, 240, 250, 260, 265, 270, 271, 272, 273 oder 274 zusammenhängenden Aminosäuren mit dem Ausgangsmolekül übereinstimmt, wobei die Protease mindestens eine der Aminosäuresubstitutionen P9T/S/H, Q62E, D101E, N130D, G166S, N187H, S216C, N238K oder Q271E an den Positionen, die den Positionen 9, 62, 101, 130, 166, 187, 216, 238 und 271 gemäß SEQ ID NO:2 entsprechen, umfasst.

4. Verfahren zur Herstellung einer Protease umfassend das Substituieren einer Aminosäure an mindestens einer der Positionen, die Positionen 9, 62, 101, 130, 166, 187, 216, 238 und 271 in SEQ ID NO:2 entsprechen, in einer Ausgangsprotease, die über ihre Gesamtlänge mindestens 90% Sequenzidentität zu der in SEQ ID NO:2 angegebenen Aminosäuresequenz aufweist, derart, dass die Protease an mindestens einer Position die Aminosäuresubstitution P9T/S/H, Q62E, D101E, N130D, G166S, N187H, S216C, N238K oder Q271E umfasst.

5. Verfahren nach Anspruch 4, ferner umfassend einen oder beide der folgenden Verfahrensschritte:
(a) Einbringen einer ein- oder mehrfachen konservativen Aminosäuresubstitution, wobei die Protease mindestens eine der Aminosäuresubstitutionen P9T/S/H, Q62E, D101E, N130D, G166S, N187H, S216C, N238K oder Q271E an den Positionen, die den Positionen 9, 62, 101, 130, 166, 187, 216, 238 und 271 gemäß SEQ ID NO:2 entsprechen, umfasst;
b) Veränderung der Aminosäuresequenz durch Fragmentierung, Deletions-, Insertions- oder Substitutionsmutagenese derart, dass die Protease eine Aminosäuresequenz umfasst, die über eine Länge von mindestens 50, 60, 70, 80, 90, 100, 110, 120, 130, 140, 150, 160, 170, 180, 190, 200, 210, 220, 230, 240, 250, 260, 265, 270, 271, 272, 273 oder 274 zusammenhängenden Aminosäuren mit dem Ausgangsmolekül übereinstimmt, wobei die Protease mindestens eine der Aminosäuresubstitutionen P9T/S/H, Q62E, D101E, N130D, G166S, N187H, S216C, N238K oder Q271E an den Positionen, die den Positionen 9, 62, 101, 130, 166, 187, 216, 238 und 271 gemäß SEQ ID NO:2 entsprechen, umfasst.

6. Nukleinsäure kodierend für eine Protease nach einem der Ansprüche 1-3 oder kodierend für eine nach einem Verfahren nach einem der Ansprüche 4 oder 5 erhältliche Protease.

7. Vektor enthaltend eine Nukleinsäure nach Anspruch 6, insbesondere ein Klonierungsvektor oder ein Expressionsvektor.

8. Nicht-menschliche Wirtszelle, die eine Nukleinsäure nach Anspruch 6 oder einen Vektor nach Anspruch 7 beinhaltet, oder die eine Protease nach einem der Ansprüche 1-3 beinhaltet, oder die eine nach einem Verfahren der Ansprüche 4 oder 5 erhältliche Protease beinhaltet.

9. Verfahren zur Herstellung einer Protease umfassend
a) Kultivieren einer Wirtszelle gemäß Anspruch 8; und
b) Isolieren der Protease aus dem Kulturmedium oder aus der Wirtszelle.

10. Mittel, insbesondere ein Wasch- oder Reinigungsmittel, **dadurch gekennzeichnet, dass** es mindestens eine Protease nach einem der Ansprüche 1-3 oder eine nach einem Verfahren der Ansprüche 4 oder 5 erhältliche Protease enthält.

11. Verfahren zur Reinigung von Textilien oder harten Oberflächen, **dadurch gekennzeichnet, dass** in mindestens einem Verfahrensschritt ein Mittel nach Anspruch 10 angewendet wird, oder dass in mindestens einem Verfahrensschritt eine Protease nach einem der Ansprüche 1-3 oder eine nach einem Verfahren der Ansprüche 4 oder 5 erhältliche Protease angewendet wird.

12. Verwendung einer Protease nach einem der Ansprüche 1-3 oder einer nach einem Verfahren der Ansprüche 4 oder 5 erhältlichen Protease in einem Wasch- oder Reinigungsmittel zur Entfernung von proteinhaltigen Anschmutzungen.

## Claims

1. A protease comprising an amino acid sequence which has, over the entire length thereof, at least 90% sequence identity with the amino acid sequence given in SEQ ID NO:2 and which has an amino acid substitution at at least one of the positions P9, Q62, D101, N130, G166, N187, S216, N238 or Q271, in each case based on the numbering according to SEQ ID NO:2, wherein the at least one amino acid substitution is selected from the group consisting of P9T/S/H, Q62E, D101E, N130D, G166S, N187H, S216C, N238K or Q271E, in each case based on the numbering according to SEQ ID NO:2.

2. The protease according to claim 1, wherein the protease has one of the following amino acid substitution variants:
(i) P9T;
(ii) Q271E;
(iii) P9S and N238K;
(iv) P9H;
(v) N187H;
(vi) S216C;
(vii) Q62E and N130D;
(viii) Q62E, D101E, N130D, G166S and Q271E; or
(ix) P9T, N187H, S216C and Q271E.

3. A protease, **characterized in that**
(a) it can be obtained from a protease according to claims 1-2 as a starting molecule by means of one or more conservative amino acid substitutions, the protease having at least one of the amino acid substitutions P9T/S/H, Q62E, D101E, N130D, G166S, N187H, S216C, N238K or Q271E at the positions corresponding to positions 9, 62, 101, 130, 166, 187, 216, 238 and 271 according to SEQ ID NO:2; and/or
(b) it can be obtained from a protease according to claims 1-2 as a starting molecule by means of fragmentation or deletion, insertion or substitution mutagenesis and comprises an amino acid sequence which matches the starting molecule over a length of at least 50, 60, 70, 80, 90, 100, 110, 120, 130, 140, 150, 160, 170, 180, 190, 200, 210, 220, 230, 240, 250, 260, 265, 270, 271, 272, 273 or 274 contiguous amino acids, wherein the protease comprises at least one of the amino acid substitutions P9T/S/H, Q62E, D101E, N130D, G166S, N187H, S216C, N238K or Q271E at the positions corresponding to positions 9, 62, 101, 130, 166, 187, 216, 238 and 271 according to SEQ ID NO:2.

4. A method for producing a protease, comprising substituting an amino acid at at least one of the positions corresponding to positions 9, 62, 101, 130, 166, 187, 216, 238 and 271 in SEQ ID NO:2 in a starting protease which has, over the entire length thereof, at least 90% sequence identity with the amino acid sequence given in SEQ ID NO:2, such that the protease comprises the amino acid substitution P9T/S/H, Q62E, D101E, N130D, G166S, N187H, S216C, N238K or Q271E at at least one position.

5. The method according to claim 4, further comprising one or both of the following method steps:
(a) introducing one or more conservative amino acid substitutions, wherein the protease comprises at least one of the amino acid substitutions P9T/S/H, Q62E, D101E, N130D, G166S, N187H, S216C, N238K or Q271E at the positions corresponding to positions 9, 62, 101, 130, 166, 187, 216, 238 and 271 according to SEQ ID NO:2;
b) altering the amino acid sequence by fragmentation or deletion, insertion or substitution mutagenesis such that the protease comprises an amino acid sequence which matches the starting molecule over a length of at least 50, 60, 70, 80, 90, 100, 110, 120, 130, 140, 150, 160, 170, 180, 190, 200, 210, 220, 230, 240, 250, 260, 265, 270, 271, 272, 273 or 274 contiguous amino acids, wherein the protease comprises at least one of the amino acid substitutions P9T/S/H, Q62E, D101E, N130D, G166S, N187H, S216C, N238K or Q271E at the positions corresponding to positions 9, 62, 101, 130, 166, 187, 216, 238 and 271 according to SEQ ID NO:2.

6. A nucleic acid coding for a protease according to one of claims 1-3 or coding for a protease that can be obtained using a method according to one of claims 4 or 5.

7. A vector containing a nucleic acid according to claim 6, in particular a cloning vector or an expression vector.

8. A non-human host cell containing a nucleic acid according to claim 6 or a vector according to claim 7, or containing a protease according to one of claims 1-3, or containing a protease that can be obtained using a method from claim 4 or 5.

9. A method for producing a protease, comprising
a) cultivating a host cell according to claim 8; and
b) isolating the protease from the culture medium or from the host cell.

10. An agent, in particular a washing or cleaning agent, **characterized in that** it contains at least one protease according to one of claims 1-3 or a protease that can be obtained using a method from claim 4 or 5.

11. A method for cleaning textiles or hard surfaces, **characterized in that** an agent according to claim 10 is used in at least one method step, or **in that** a protease according to one of claims 1-3 or a protease that can be obtained using a method from claim 4 or 5 is used in at least one method step.

12. The use of a protease according to one of claims 1-3 or of a protease that can be obtained using a method from claim 4 or 5, in a washing or cleaning agent for removing protein-containing stains.

## Revendications

1. Protéase comprenant une séquence d'acides aminés qui présente, sur toute sa longueur, au moins 90 % d'identité de séquence avec la séquence d'acides aminés indiquée dans SEQ ID NO: 2, et qui présente une substitution d'acides aminés à au moins l'une des positions P9, Q62, D101, N130, G166, N187, S216, N238 ou Q271, chacune étant basée sur la numérotation selon SEQ ID NO: 2, l'au moins une substitution d'acides aminés étant choisie dans le groupe constitué par P9T/S/H, Q62E, D101E, N130D, G166S, N187H, S216C, N238K ou Q271E, chacune étant basée sur la numérotation selon SEQ ID NO: 2.

2. Protéase selon la revendication 1, la protéase présentant l'un des variants de substitution d'acides aminés suivants :
(i) P9T ;
(ii) Q271E ;
(iii) P9S et N238K ;
(iv) P9H ;
(v) N187H ;
(vi) S216C ;
(vii) Q62E et N130D ;
(viii) Q62E, D101E, N130D, G166S et Q271E ; ou
(ix) P9T, N187H, S216C et Q271E.

3. Protéase, **caractérisée**
(a) **en ce qu'**elle peut être obtenue à partir d'une protéase selon la revendication 1 à 2 en tant que molécule de départ, par substitution conservatrice unique ou multiple d'acides aminés, la protéase présentant au moins l'une des substitutions d'acides aminés P9T/S/H, Q62E, D101E, N130D, G166S, N187H, S216C, N238K ou Q271E aux positions correspondant aux positions 9, 62, 101, 130, 166, 187, 216, 238 et 271 selon SEQ ID NO: 2 ; et/ou
(b) **en ce qu'**elle peut être obtenue à partir d'une protéase selon la revendication 1 à 2 en tant que molécule de départ, par fragmentation, mutagenèse par délétion, insertion ou substitution, et en ce qu'elle comprend une séquence d'acides aminés qui correspond à la molécule de départ sur une longueur d'au moins 50, 60, 70, 80, 90, 100, 110, 120, 130, 140, 150, 160, 170, 180, 190, 200, 210, 220, 230, 240, 250, 260, 265, 270, 271, 272, 273 ou 274 acides aminés contigus, la protéase comprenant au moins l'une des substitutions d'acides aminés P9T/S/H, Q62E, D101E, N130D, G166S, N187H, S216C, N238K ou Q271E aux positions correspondant aux positions 9, 62,101, 130,166,187, 216, 238 et 271 selon SEQ ID NO: 2.

4. Procédé de production d'une protéase comprenant la substitution d'un acide aminé à au moins l'une des positions correspondant aux positions 9, 62, 101, 130, 166, 187, 216, 238 et 271 dans SEQ ID NO: 2 dans une protéase de départ, laquelle présente, sur toute sa longueur, au moins 90 % d'identité de séquence avec la séquence d'acides aminés indiquée dans SEQ ID NO: 2, de telle sorte que la protéase comprend, à au moins une position, la substitution d'acides aminés P9T/S/H, Q62E, D101E, N130D, G166S, N187H, S216C, N238K ou Q271E.

5. Procédé selon la revendication 4, comprenant en outre une ou les deux étapes de procédé suivantes :
(a) introduction d'une substitution conservatrice unique ou multiple d'acides aminés, la protéase comprenant au moins l'une des substitutions d'acides aminés P9T/S/H, Q62E, D101E, N130D, G166S, N187H, S216C, N238K ou Q271E aux positions correspondant aux positions 9, 62, 101, 130, 166, 187, 216, 238 et 271 selon SEQ ID NO: 2;
b) modification de la séquence d'acides aminés par fragmentation, mutagenèse par délétion, insertion ou substitution, de telle sorte que la protéase comprend une séquence d'acides aminés qui correspond à la molécule de départ sur une longueur d'au moins 50, 60, 70, 80, 90, 100, 110, 120, 130, 140, 150, 160, 170, 180, 190, 200, 210, 220, 230, 240, 250, 260, 265, 270, 271,272, 273 ou 274 acides aminés contigus, la protéase comprenant au moins l'une des substitutions d'acides aminés P9T/S/H, Q62E, D101E, N130D, G166S, N187H, S216C, N238K ou Q271E aux positions correspondant aux positions 9, 62, 101, 130, 166, 187, 216, 238 et 271 selon SEQ ID NO: 2.

6. Acide nucléique codant pour une protéase selon l'une des revendications 1 à 3 ou codant pour une protéase pouvant être obtenue selon un procédé selon l'une des revendications 4 ou 5.

7. Vecteur contenant un acide nucléique selon la revendication 6, en particulier un vecteur de clonage ou un vecteur d'expression.

8. Cellule hôte non humaine qui contient un acide nucléique selon la revendication 6 ou un vecteur selon la revendication 7, ou qui contient une protéase selon l'une des revendications 1 à 3, ou qui contient une protéase pouvant être obtenue selon un procédé des revendications 4 ou 5.

9. Procédé de préparation d'une protéase, comprenant
a) la culture d'une cellule hôte selon la revendication 8 ; et
b) l'isolement de la protéase à partir du milieu de culture ou de la cellule hôte.

10. Agent, en particulier agent de lavage ou de nettoyage, **caractérisé en ce qu'**il contient au moins une protéase selon l'une des revendications 1 à 3 ou une protéase pouvant être obtenue selon un procédé des revendications 4 ou 5.

11. Procédé de nettoyage de textiles ou de surfaces dures, **caractérisé en ce qu'**un agent selon la revendication 10 est utilisé dans au moins une étape de procédé, ou **en ce qu'**une protéase selon l'une des revendications 1 à 3 ou une protéase pouvant être obtenue selon un procédé des revendications 4 ou 5 est utilisée dans au moins une étape de procédé.

12. Utilisation d'une protéase selon l'une des revendications 1 à 3 ou d'une protéase pouvant être obtenue selon un procédé des revendications 4 ou 5 dans un agent de lavage ou de nettoyage pour éliminer des salissures contenant des protéines.


### SEQUENCE LISTING

<110> Henkel AG & Co. KGaA
<120> Proteasen mit verbesserte Enzymstabilität in Waschmittel
<130> PT033537PCT
<150> 102016204815.5
   <151> 2016-03-23
<160> 11
<170> PatentIn version 3.5
<210> 1
   <211> 825
   <212> DNA
   <213> Bacillus pumilus
<400> 1
   gcacaaaccg tcccttatgg aatccctcaa atcaaagctc cagctgtaca cgctcaaggt      60
   tataaaggtg ctaatgtcaa agtagctgtc cttgatactg gaatccacgc tgcacatcct     120
   gacttaaatg ttgcaggcgg tgccagcttc gtcccttcag agccaaatgc cacccaagac     180
   tttcaatcac atggaactca cgtagccgga accattgctg cccttgataa cacaattggt     240
   gttcttgggg tcgctccaag tgcttcccta tatgctgtta aagtattaga ccgcaatggc     300
   gacggacaat acagctggat cattagcggt attgaatggg ctgtagccaa taacatggat     360
   gtcatcaata tgagcttagg tggaccaaac ggttcaacag cgcttaaaaa tgccgttgat     420
   acagcaaata accgcggagt cgttgttgtg gcggccgcag gtaattcagg ttccactggc     480
   tctacaagta cagttggcta tccagcaaaa tatgattcta caattgccgt tgccaatgta     540
   aacagcagca atgtcagaaa ctcgtcttcc agcgcaggtc ctgaattaga tgtttctgca     600
   cctggtactt ctattttaag tacagtacca agcagtggat acacatctta tactggaact     660
   tctatggcgt ctcctcatgt agcaggagca gcagcgctta ttctttctaa aaatccgaac     720
   ctatcaaatt cacaggttcg ccagcgctta gaaaacacgg caacaccgct tggtaactcc     780
   ttctattacg gaaaagggtt aatcaacgct caagcggctt ctaac                     825
<210> 2
   <211> 275
   <212> PRT
   <213> Bacillus pumilus
<400> 2
   Ala Gln Thr Val Pro Tyr Gly Ile Pro Gln Ile Lys Ala Pro Ala Val
   1               5                   10                  15
   His Ala Gln Gly Tyr Lys Gly Ala Asn Val Lys Val Ala Val Leu Asp
               20                  25                  30
   Thr Gly Ile His Ala Ala His Pro Asp Leu Asn Val Ala Gly Gly Ala
           35                  40                  45
   Ser Phe Val Pro Ser Glu Pro Asn Ala Thr Gln Asp Phe Gln Ser His
       50                  55                  60
   Gly Thr His Val Ala Gly Thr Ile Ala Ala Leu Asp Asn Thr Ile Gly
   65                  70                  75                  80
   Val Leu Gly Val Ala Pro Ser Ala Ser Leu Tyr Ala Val Lys Val Leu
                   85                  90                  95
   Asp Arg Asn Gly Asp Gly Gln Tyr Ser Trp Ile Ile Ser Gly Ile Glu
               100                 105                 110
   Trp Ala Val Ala Asn Asn Met Asp Val Ile Asn Met Ser Leu Gly Gly
           115                 120                 125
   Pro Asn Gly Ser Thr Ala Leu Lys Asn Ala Val Asp Thr Ala Asn Asn
       130                 135                 140
   Arg Gly Val Val Val Val Ala Ala Ala Gly Asn Ser Gly Ser Thr Gly
   145                 150                 155                 160
   Ser Thr Ser Thr Val Gly Tyr Pro Ala Lys Tyr Asp Ser Thr Ile Ala
                   165                 170                 175
   Val Ala Asn Val Asn Ser Ser Asn Val Arg Asn Ser Ser Ser Ser Ala
               180                 185                 190
   Gly Pro Glu Leu Asp Val Ser Ala Pro Gly Thr Ser Ile Leu Ser Thr
           195                 200                 205
   Val Pro Ser Ser Gly Tyr Thr Ser Tyr Thr Gly Thr Ser Met Ala Ser
       210                 215                 220
   Pro His Val Ala Gly Ala Ala Ala Leu Ile Leu Ser Lys Asn Pro Asn
   225                 230                 235                 240
   Leu Ser Asn Ser Gln Val Arg Gln Arg Leu Glu Asn Thr Ala Thr Pro
                   245                 250                 255
   Leu Gly Asn Ser Phe Tyr Tyr Gly Lys Gly Leu Ile Asn Ala Gln Ala
               260                 265                 270
   Ala Ser Asn
   275
<210> 3
   <211> 275
   <212> PRT
   <213> Artificial Sequence
<220>
   <223> Bacillus pumilus protease mutant
<400> 3
   Ala Gln Thr Val Pro Tyr Gly Ile Thr Gln Ile Lys Ala Pro Ala Val
   1               5                   10                  15
   His Ala Gln Gly Tyr Lys Gly Ala Asn Val Lys Val Ala Val Leu Asp
               20                  25                  30
   Thr Gly Ile His Ala Ala His Pro Asp Leu Asn Val Ala Gly Gly Ala
           35                  40                  45
   Ser Phe Val Pro Ser Glu Pro Asn Ala Thr Gln Asp Phe Gln Ser His
       50                  55                  60
   Gly Thr His Val Ala Gly Thr Ile Ala Ala Leu Asp Asn Thr Ile Gly
   65                  70                  75                  80
   Val Leu Gly Val Ala Pro Ser Ala Ser Leu Tyr Ala Val Lys Val Leu
                   85                  90                  95
   Asp Arg Asn Gly Asp Gly Gln Tyr Ser Trp Ile Ile Ser Gly Ile Glu
               100                 105                 110
   Trp Ala Val Ala Asn Asn Met Asp Val Ile Asn Met Ser Leu Gly Gly
           115                 120                 125
   Pro Asn Gly Ser Thr Ala Leu Lys Asn Ala Val Asp Thr Ala Asn Asn
       130                 135                 140
   Arg Gly Val Val Val Val Ala Ala Ala Gly Asn Ser Gly Ser Thr Gly
   145                 150                 155                 160
   Ser Thr Ser Thr Val Gly Tyr Pro Ala Lys Tyr Asp Ser Thr Ile Ala
                   165                 170                 175
   Val Ala Asn Val Asn Ser Ser Asn Val Arg Asn Ser Ser Ser Ser Ala
               180                 185                 190
   Gly Pro Glu Leu Asp Val Ser Ala Pro Gly Thr Ser Ile Leu Ser Thr
           195                 200                 205
   Val Pro Ser Ser Gly Tyr Thr Ser Tyr Thr Gly Thr Ser Met Ala Ser
       210                 215                 220
   Pro His Val Ala Gly Ala Ala Ala Leu Ile Leu Ser Lys Asn Pro Asn
   225                 230                 235                 240
   Leu Ser Asn Ser Gln Val Arg Gln Arg Leu Glu Asn Thr Ala Thr Pro
                   245                 250                 255
   Leu Gly Asn Ser Phe Tyr Tyr Gly Lys Gly Leu Ile Asn Ala Gln Ala
               260                 265                 270
   Ala Ser Asn
           275
<210> 4
   <211> 275
   <212> PRT
   <213> Artificial Sequence
<220>
   <223> Bacillus pumilus protease mutant
<400> 4
   Ala Gln Thr Val Pro Tyr Gly Ile Pro Gln Ile Lys Ala Pro Ala Val
   1               5                   10                  15
   His Ala Gln Gly Tyr Lys Gly Ala Asn Val Lys Val Ala Val Leu Asp
               20                  25                  30
   Thr Gly Ile His Ala Ala His Pro Asp Leu Asn Val Ala Gly Gly Ala
           35                  40                  45
   Ser Phe Val Pro Ser Glu Pro Asn Ala Thr Gln Asp Phe Gln Ser His
       50                  55                  60
   Gly Thr His Val Ala Gly Thr Ile Ala Ala Leu Asp Asn Thr Ile Gly
   65                  70                  75                  80
   Val Leu Gly Val Ala Pro Ser Ala Ser Leu Tyr Ala Val Lys Val Leu
                   85                  90                  95
   Asp Arg Asn Gly Asp Gly Gln Tyr Ser Trp Ile Ile Ser Gly Ile Glu
               100                 105                 110
   Trp Ala Val Ala Asn Asn Met Asp Val Ile Asn Met Ser Leu Gly Gly
           115                 120                 125
   Pro Asn Gly Ser Thr Ala Leu Lys Asn Ala Val Asp Thr Ala Asn Asn
       130                 135                 140
   Arg Gly Val Val Val Val Ala Ala Ala Gly Asn Ser Gly Ser Thr Gly
   145                 150                 155                 160
   Ser Thr Ser Thr Val Gly Tyr Pro Ala Lys Tyr Asp Ser Thr Ile Ala
                   165                 170                 175
   Val Ala Asn Val Asn Ser Ser Asn Val Arg Asn Ser Ser Ser Ser Ala
               180                 185                 190
   Gly Pro Glu Leu Asp Val Ser Ala Pro Gly Thr Ser Ile Leu Ser Thr
           195                 200                 205
   Val Pro Ser Ser Gly Tyr Thr Ser Tyr Thr Gly Thr Ser Met Ala Ser
       210                 215                 220
   Pro His Val Ala Gly Ala Ala Ala Leu Ile Leu Ser Lys Asn Pro Asn
   225                 230                 235                 240
   Leu Ser Asn Ser Gln Val Arg Gln Arg Leu Glu Asn Thr Ala Thr Pro
                   245                 250                 255
   Leu Gly Asn Ser Phe Tyr Tyr Gly Lys Gly Leu Ile Asn Ala Glu Ala
               260                 265                 270
   Ala Ser Asn
           275
<210> 5
   <211> 275
   <212> PRT
   <213> Artificial Sequence
<220>
   <223> Bacillus pumilus protease mutant
<400> 5
   Ala Gln Thr Val Pro Tyr Gly Ile Ser Gln Ile Lys Ala Pro Ala Val
   1               5                   10                  15
   His Ala Gln Gly Tyr Lys Gly Ala Asn Val Lys Val Ala Val Leu Asp
               20                  25                  30
   Thr Gly Ile His Ala Ala His Pro Asp Leu Asn Val Ala Gly Gly Ala
           35                  40                  45
   Ser Phe Val Pro Ser Glu Pro Asn Ala Thr Gln Asp Phe Gln Ser His
       50                  55                  60
   Gly Thr His Val Ala Gly Thr Ile Ala Ala Leu Asp Asn Thr Ile Gly
   65                  70                  75                  80
   Val Leu Gly Val Ala Pro Ser Ala Ser Leu Tyr Ala Val Lys Val Leu
                   85                  90                  95
   Asp Arg Asn Gly Asp Gly Gln Tyr Ser Trp Ile Ile Ser Gly Ile Glu
               100                 105                 110
   Trp Ala Val Ala Asn Asn Met Asp Val Ile Asn Met Ser Leu Gly Gly
           115                 120                 125
   Pro Asn Gly Ser Thr Ala Leu Lys Asn Ala Val Asp Thr Ala Asn Asn
       130                 135                 140
   Arg Gly Val Val Val Val Ala Ala Ala Gly Asn Ser Gly Ser Thr Gly
   145                 150                 155                 160
   Ser Thr Ser Thr Val Gly Tyr Pro Ala Lys Tyr Asp Ser Thr Ile Ala
                   165                 170                 175
   Val Ala Asn Val Asn Ser Ser Asn Val Arg Asn Ser Ser Ser Ser Ala
               180                 185                 190
   Gly Pro Glu Leu Asp Val Ser Ala Pro Gly Thr Ser Ile Leu Ser Thr
           195                 200                 205
   Val Pro Ser Ser Gly Tyr Thr Ser Tyr Thr Gly Thr Ser Met Ala Ser
       210                 215                 220
   Pro His Val Ala Gly Ala Ala Ala Leu Ile Leu Ser Lys Lys Pro Asn
   225                 230                 235                 240
   Leu Ser Asn Ser Gln Val Arg Gln Arg Leu Glu Asn Thr Ala Thr Pro
                   245                 250                 255
   Leu Gly Asn Ser Phe Tyr Tyr Gly Lys Gly Leu Ile Asn Ala Gln Ala
               260                 265                 270
   Ala Ser Asn
           275
<210> 6
   <211> 275
   <212> PRT
   <213> Artificial Sequence
<220>
   <223> Bacillus pumilus protease mutant
<400> 6
   Ala Gln Thr Val Pro Tyr Gly Ile His Gln Ile Lys Ala Pro Ala Val
   1               5                   10                  15
   His Ala Gln Gly Tyr Lys Gly Ala Asn Val Lys Val Ala Val Leu Asp
               20                  25                  30
   Thr Gly Ile His Ala Ala His Pro Asp Leu Asn Val Ala Gly Gly Ala
           35                  40                  45
   Ser Phe Val Pro Ser Glu Pro Asn Ala Thr Gln Asp Phe Gln Ser His
       50                  55                  60
   Gly Thr His Val Ala Gly Thr Ile Ala Ala Leu Asp Asn Thr Ile Gly
   65                  70                  75                  80
   Val Leu Gly Val Ala Pro Ser Ala Ser Leu Tyr Ala Val Lys Val Leu
                   85                  90                  95
   Asp Arg Asn Gly Asp Gly Gln Tyr Ser Trp Ile Ile Ser Gly Ile Glu
               100                 105                 110
   Trp Ala Val Ala Asn Asn Met Asp Val Ile Asn Met Ser Leu Gly Gly
           115                 120                 125
   Pro Asn Gly Ser Thr Ala Leu Lys Asn Ala Val Asp Thr Ala Asn Asn
       130                 135                 140
   Arg Gly Val Val Val Val Ala Ala Ala Gly Asn Ser Gly Ser Thr Gly
   145                 150                 155                 160
   Ser Thr Ser Thr Val Gly Tyr Pro Ala Lys Tyr Asp Ser Thr Ile Ala
                   165                 170                 175
   Val Ala Asn Val Asn Ser Ser Asn Val Arg Asn Ser Ser Ser Ser Ala
               180                 185                 190
   Gly Pro Glu Leu Asp Val Ser Ala Pro Gly Thr Ser Ile Leu Ser Thr
           195                 200                 205
   Val Pro Ser Ser Gly Tyr Thr Ser Tyr Thr Gly Thr Ser Met Ala Ser
       210                 215                 220
   Pro His Val Ala Gly Ala Ala Ala Leu Ile Leu Ser Lys Asn Pro Asn
   225                 230                 235                 240
   Leu Ser Asn Ser Gln Val Arg Gln Arg Leu Glu Asn Thr Ala Thr Pro
                   245                 250                 255
   Leu Gly Asn Ser Phe Tyr Tyr Gly Lys Gly Leu Ile Asn Ala Gln Ala
               260                 265                 270
   Ala Ser Asn
           275
<210> 7
   <211> 275
   <212> PRT
   <213> Artificial Sequence
<220>
   <223> Bacillus pumilus protease mutant
<400> 7
   Ala Gln Thr Val Pro Tyr Gly Ile Pro Gln Ile Lys Ala Pro Ala Val
   1               5                   10                  15
   His Ala Gln Gly Tyr Lys Gly Ala Asn Val Lys Val Ala Val Leu Asp
               20                  25                  30
   Thr Gly Ile His Ala Ala His Pro Asp Leu Asn Val Ala Gly Gly Ala
           35                  40                  45
   Ser Phe Val Pro Ser Glu Pro Asn Ala Thr Gln Asp Phe Gln Ser His
       50                  55                  60
   Gly Thr His Val Ala Gly Thr Ile Ala Ala Leu Asp Asn Thr Ile Gly
   65                  70                  75                  80
   Val Leu Gly Val Ala Pro Ser Ala Ser Leu Tyr Ala Val Lys Val Leu
                   85                  90                  95
   Asp Arg Asn Gly Asp Gly Gln Tyr Ser Trp Ile Ile Ser Gly Ile Glu
               100                 105                 110
   Trp Ala Val Ala Asn Asn Met Asp Val Ile Asn Met Ser Leu Gly Gly
           115                 120                 125
   Pro Asn Gly Ser Thr Ala Leu Lys Asn Ala Val Asp Thr Ala Asn Asn
       130                 135                 140
   Arg Gly Val Val Val Val Ala Ala Ala Gly Asn Ser Gly Ser Thr Gly
   145                 150                 155                 160
   Ser Thr Ser Thr Val Gly Tyr Pro Ala Lys Tyr Asp Ser Thr Ile Ala
                   165                 170                 175
   Val Ala Asn Val Asn Ser Ser Asn Val Arg His Ser Ser Ser Ser Ala
               180                 185                 190
   Gly Pro Glu Leu Asp Val Ser Ala Pro Gly Thr Ser Ile Leu Ser Thr
           195                 200                 205
   Val Pro Ser Ser Gly Tyr Thr Ser Tyr Thr Gly Thr Ser Met Ala Ser
       210                 215                 220
   Pro His Val Ala Gly Ala Ala Ala Leu Ile Leu Ser Lys Asn Pro Asn
   225                 230                 235                 240
   Leu Ser Asn Ser Gln Val Arg Gln Arg Leu Glu Asn Thr Ala Thr Pro
                   245                 250                 255
   Leu Gly Asn Ser Phe Tyr Tyr Gly Lys Gly Leu Ile Asn Ala Gln Ala
               260                 265                 270
   Ala Ser Asn
           275
<210> 8
   <211> 275
   <212> PRT
   <213> Artificial Sequence
<220>
   <223> Bacillus pumilus protease mutant
<400> 8
   Ala Gln Thr Val Pro Tyr Gly Ile Pro Gln Ile Lys Ala Pro Ala Val
   1               5                   10                  15
   His Ala Gln Gly Tyr Lys Gly Ala Asn Val Lys Val Ala Val Leu Asp
               20                  25                  30
   Thr Gly Ile His Ala Ala His Pro Asp Leu Asn Val Ala Gly Gly Ala
           35                  40                  45
   Ser Phe Val Pro Ser Glu Pro Asn Ala Thr Gln Asp Phe Gln Ser His
       50                  55                  60
   Gly Thr His Val Ala Gly Thr Ile Ala Ala Leu Asp Asn Thr Ile Gly
   65                  70                  75                  80
   Val Leu Gly Val Ala Pro Ser Ala Ser Leu Tyr Ala Val Lys Val Leu
                   85                  90                  95
   Asp Arg Asn Gly Asp Gly Gln Tyr Ser Trp Ile Ile Ser Gly Ile Glu
               100                 105                 110
   Trp Ala Val Ala Asn Asn Met Asp Val Ile Asn Met Ser Leu Gly Gly
           115                 120                 125
   Pro Asn Gly Ser Thr Ala Leu Lys Asn Ala Val Asp Thr Ala Asn Asn
       130                 135                 140
   Arg Gly Val Val Val Val Ala Ala Ala Gly Asn Ser Gly Ser Thr Gly
   145                 150                 155                 160
   Ser Thr Ser Thr Val Gly Tyr Pro Ala Lys Tyr Asp Ser Thr Ile Ala
                   165                 170                 175
   Val Ala Asn Val Asn Ser Ser Asn Val Arg Asn Ser Ser Ser Ser Ala
               180                 185                 190
   Gly Pro Glu Leu Asp Val Ser Ala Pro Gly Thr Ser Ile Leu Ser Thr
           195                 200                 205
   Val Pro Ser Ser Gly Tyr Thr Cys Tyr Thr Gly Thr Ser Met Ala Ser
       210                 215                 220
   Pro His Val Ala Gly Ala Ala Ala Leu Ile Leu Ser Lys Asn Pro Asn
   225                 230                 235                 240
   Leu Ser Asn Ser Gln Val Arg Gln Arg Leu Glu Asn Thr Ala Thr Pro
                   245                 250                 255
   Leu Gly Asn Ser Phe Tyr Tyr Gly Lys Gly Leu Ile Asn Ala Gln Ala
               260                 265                 270
   Ala Ser Asn
           275
<210> 9
   <211> 275
   <212> PRT
   <213> Artificial Sequence
<220>
   <223> Bacillus pumilus protease mutant
<400> 9
   Ala Gln Thr Val Pro Tyr Gly Ile Pro Gln Ile Lys Ala Pro Ala Val
   1               5                   10                  15
   His Ala Gln Gly Tyr Lys Gly Ala Asn Val Lys Val Ala Val Leu Asp
               20                  25                  30
   Thr Gly Ile His Ala Ala His Pro Asp Leu Asn Val Ala Gly Gly Ala
           35                  40                  45
   Ser Phe Val Pro Ser Glu Pro Asn Ala Thr Gln Asp Phe Glu Ser His
       50                  55                  60
   Gly Thr His Val Ala Gly Thr Ile Ala Ala Leu Asp Asn Thr Ile Gly
   65                  70                  75                  80
   Val Leu Gly Val Ala Pro Ser Ala Ser Leu Tyr Ala Val Lys Val Leu
                   85                  90                  95
   Asp Arg Asn Gly Asp Gly Gln Tyr Ser Trp Ile Ile Ser Gly Ile Glu
               100                 105                 110
   Trp Ala Val Ala Asn Asn Met Asp Val Ile Asn Met Ser Leu Gly Gly
           115                 120                 125
   Pro Asp Gly Ser Thr Ala Leu Lys Asn Ala Val Asp Thr Ala Asn Asn
       130                 135                 140
   Arg Gly Val Val Val Val Ala Ala Ala Gly Asn Ser Gly Ser Thr Gly
   145                 150                 155                 160
   Ser Thr Ser Thr Val Gly Tyr Pro Ala Lys Tyr Asp Ser Thr Ile Ala
                   165                 170                 175
   Val Ala Asn Val Asn Ser Ser Asn Val Arg Asn Ser Ser Ser Ser Ala
               180                 185                 190
   Gly Pro Glu Leu Asp Val Ser Ala Pro Gly Thr Ser Ile Leu Ser Thr
           195                 200                 205
   Val Pro Ser Ser Gly Tyr Thr Ser Tyr Thr Gly Thr Ser Met Ala Ser
       210                 215                 220
   Pro His Val Ala Gly Ala Ala Ala Leu Ile Leu Ser Lys Asn Pro Asn
   225                 230                 235                 240
   Leu Ser Asn Ser Gln Val Arg Gln Arg Leu Glu Asn Thr Ala Thr Pro
                   245                 250                 255
   Leu Gly Asn Ser Phe Tyr Tyr Gly Lys Gly Leu Ile Asn Ala Gln Ala
   260 265 270
   Ala Ser Asn
   275
<210> 10
   <211> 275
   <212> PRT
   <213> Artificial Sequence
<220>
   <223> Bacillus pumilus protease mutant
<400> 10
   Ala Gln Thr Val Pro Tyr Gly Ile Pro Gln Ile Lys Ala Pro Ala Val
   1 5 10 15
   His Ala Gln Gly Tyr Lys Gly Ala Asn Val Lys Val Ala Val Leu Asp
   20 25 30
   Thr Gly Ile His Ala Ala His Pro Asp Leu Asn Val Ala Gly Gly Ala
   35 40 45
   Ser Phe Val Pro Ser Glu Pro Asn Ala Thr Gln Asp Phe Glu Ser His
   50 55 60
   Gly Thr His Val Ala Gly Thr Ile Ala Ala Leu Asp Asn Thr Ile Gly
   65 70 75 80
   Val Leu Gly Val Ala Pro Ser Ala Ser Leu Tyr Ala Val Lys Val Leu
   85 90 95
   Asp Arg Asn Gly Glu Gly Gln Tyr Ser Trp Ile Ile Ser Gly Ile Glu
   100 105 110
   Trp Ala Val Ala Asn Asn Met Asp Val Ile Asn Met Ser Leu Gly Gly
   115 120 125
   Pro Asp Gly Ser Thr Ala Leu Lys Asn Ala Val Asp Thr Ala Asn Asn
   130 135 140
   Arg Gly Val Val Val Val Ala Ala Ala Gly Asn Ser Gly Ser Thr Gly
   145 150 155 160
   Ser Thr Ser Thr Val Ser Tyr Pro Ala Lys Tyr Asp Ser Thr Ile Ala
   165 170 175
   Val Ala Asn Val Asn Ser Ser Asn Val Arg Asn Ser Ser Ser Ser Ala
   180 185 190
   Gly Pro Glu Leu Asp Val Ser Ala Pro Gly Thr Ser Ile Leu Ser Thr
   195 200 205
   Val Pro Ser Ser Gly Tyr Thr Ser Tyr Thr Gly Thr Ser Met Ala Ser
   210 215 220
   Pro His Val Ala Gly Ala Ala Ala Leu Ile Leu Ser Lys Asn Pro Asn
   225 230 235 240
   Leu Ser Asn Ser Gln Val Arg Gln Arg Leu Glu Asn Thr Ala Thr Pro
   245 250 255
   Leu Gly Asn Ser Phe Tyr Tyr Gly Lys Gly Leu Ile Asn Ala Glu Ala
   260 265 270
   Ala Ser Asn
   275
<210> 11
   <211> 275
   <212> PRT
   <213> Artificial Sequence
<220>
   <223> Bacillus pumilus protease mutant
<400> 11
   Ala Gln Thr Val Pro Tyr Gly Ile Thr Gln Ile Lys Ala Pro Ala Val
   1 5 10 15
   His Ala Gln Gly Tyr Lys Gly Ala Asn Val Lys Val Ala Val Leu Asp
   20 25 30
   Thr Gly Ile His Ala Ala His Pro Asp Leu Asn Val Ala Gly Gly Ala
   35 40 45
   Ser Phe Val Pro Ser Glu Pro Asn Ala Thr Gln Asp Phe Gln Ser His
   50 55 60
   Gly Thr His Val Ala Gly Thr Ile Ala Ala Leu Asp Asn Thr Ile Gly
   65 70 75 80
   Val Leu Gly Val Ala Pro Ser Ala Ser Leu Tyr Ala Val Lys Val Leu
   85 90 95
   Asp Arg Asn Gly Asp Gly Gln Tyr Ser Trp Ile Ile Ser Gly Ile Glu
               100                 105                 110
   Trp Ala Val Ala Asn Asn Met Asp Val Ile Asn Met Ser Leu Gly Gly
           115                 120                 125
   Pro Asn Gly Ser Thr Ala Leu Lys Asn Ala Val Asp Thr Ala Asn Asn
       130                 135                 140
   Arg Gly Val Val Val Val Ala Ala Ala Gly Asn Ser Gly Ser Thr Gly
   145                 150                 155                 160
   Ser Thr Ser Thr Val Gly Tyr Pro Ala Lys Tyr Asp Ser Thr Ile Ala
                   165                 170                 175
   Val Ala Asn Val Asn Ser Ser Asn Val Arg His Ser Ser Ser Ser Ala
               180                 185                 190
   Gly Pro Glu Leu Asp Val Ser Ala Pro Gly Thr Ser Ile Leu Ser Thr
           195                 200                 205
   Val Pro Ser Ser Gly Tyr Thr Cys Tyr Thr Gly Thr Ser Met Ala Ser
       210                 215                 220
   Pro His Val Ala Gly Ala Ala Ala Leu Ile Leu Ser Lys Asn Pro Asn
   225                 230                 235                 240
   Leu Ser Asn Ser Gln Val Arg Gln Arg Leu Glu Asn Thr Ala Thr Pro
                   245                 250                 255
   Leu Gly Asn Ser Phe Tyr Tyr Gly Lys Gly Leu Ile Asn Ala Glu Ala
               260                 265                 270
   Ala Ser Asn
           275